(19) Europäisches Patentamt
European Patent Office
Office européen des brevets

(11) Veröffentlichungsnummer: **0 442 077 B1**

(12) **EUROPÄISCHE PATENTSCHRIFT**

(45) Veröffentlichungstag der Patentschrift: **08.11.95**

(21) Anmeldenummer: **90123878.2**

(22) Anmeldetag: **12.12.90**

(51) Int. Cl.6: **C07D 207/38**, C07D 471/04, C07D 487/04, C07D 513/04, A01N 43/36, A01N 43/90, A01N 57/24, //(C07D471/04, 221:00,209:00),(C07D487/04, 209:00,209:00)

# (54) 3-Aryl-pyrrolidin-2,4-dion-Derivate als Insektizide und Herbizide.

(30) Priorität: **14.02.90 DE 4004496**

(43) Veröffentlichungstag der Anmeldung:
**21.08.91 Patentblatt 91/34**

(45) Bekanntmachung des Hinweises auf die Patenterteilung:
**08.11.95 Patentblatt 95/45**

(84) Benannte Vertragsstaaten:
**BE CH DE FR GB IT LI NL**

(56) Entgegenhaltungen:
**EP-A- 0 262 399**
**EP-A- 0 355 599**
**EP-A- 0 377 893**
**EP-A- 0 415 211**
**WO-A-88/04652**

(73) Patentinhaber: **BAYER AG**
**D-51368 Leverkusen (DE)**

(72) Erfinder: **Bertram, Heinz Jürgen, Dr.**
**Nesselroderstrasse 27**
**W-5300 Bonn (DE)**
Erfinder: **Fischer, Reiner, Dr.**
**Nelly-Sachs-Strasse 23**
**W-4019 Monheim 2 (DE)**
Erfinder: **Krüger, Bernd-Wieland, Dr.**
**Unterboschbach 19**
**W-5060 Bergisch Gladbach (DE)**
Erfinder: **Erdelen, Christoph, Dr.**
**Unterbüscherhof 22**
**W-5653 Leichlingen 1 (DE)**
Erfinder: **Lürssen, Klaus, Dr.**
**August-Kierspel-Strasse 145**
**W-5060 Bergisch Gladbach (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**LIEBIGS ANNALEN DER CHEMIE, Nr. 5, 1985, Seiten 1095-1098, Weinheim, DE; R.SCHMIERER et al.: "Cyclisierung vin N-Acylalanin- und N-Acylglycinestern"**


Erfinder: **Schmidt, Robert R., Dr.**
**Im Waldwinkel 110**
**W-5060 Bergisch Gladbach (DE)**
Erfinder: **Santel, Hans-Joachim, Dr.**
**Grünstrasse 9a**
**W-5090 Leverkusen 1 (DE)**

## Beschreibung

Die Erfindung betrifft neue 3-Aryl-pyrrolidin-2,4-dion-Derivate, mehrere Verfahren zu ihrer Herstellung und ihre Verwendung als Insektizide, Akarizide und Herbizide.

Von 3-Acyl-pyrrolidin-2,4-dionen sind pharmazeutische Eigenschaften vorbeschrieben (S. Suzuki et. al. Chem. Pharm. Bull. 15 1120 (1967)). Weiterhin wurden N-Phenylpyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist.

In EP-A-0 355 599, EP-A-0 377 893 und EP-A-0 415 211 werden strukturell ähnliche 3-Aryl-pyrrolidin-2,4-dione mit insektiziden, akariziden, herbiziden und zum Teil auch fungiziden Eigenschaften beschrieben.

Es wurden nun neue 3-Arylpyrrolidin-2,4-dion-Derivate gefunden, die durch die Formel (I) dargestellt sind,

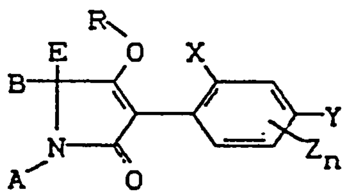

(I)

in welcher

| | |
|---|---|
| A | für gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann oder gegebenenfalls durch Halogen, $C_1$-$C_6$-Alkyl, $C_1$-$C_6$-Haloalkyl, $C_1$-$C_6$-Alkoxy, Nitro substituiertes Aryl-$C_1$-$C_6$-alkyl steht, |
| B und E | unabhängig voneinander für Wasserstoff, geradkettiges oder verzweigtes $C_1$-$C_{12}$-Alkyl, $C_1$-$C_8$-Alkoxyalkyl stehen, oder |
| A und B | mit dem Stickstoff- bzw. Kohlenstoffatom, an das sie gebunden sind, einen 4 bis 8-gliedrigen Zyklus bilden, der durch ein Schwefelatom, eine Sulfoxid- oder eine Sulfonylgruppe unterbrochen sein kann, |
| X | für $C_1$-$C_6$-Alkyl, Halogen oder $C_1$-$C_6$-Alkoxy steht, |
| Y | für Wasserstoff, $C_1$-$C_6$-Alkyl, Halogen, $C_1$-$C_6$-Alkoxy, $C_1$-$C_3$-Halogenalkyl steht, |
| Z | für $C_1$-$C_6$-Alkyl, Halogen, $C_1$-$C_6$-Alkoxy steht, |
| n | für eine Zahl von 0 - 3 steht, |
| R | für die Gruppen |

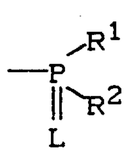 (a) $-SO_2-R^3$ (b)

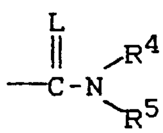 (c) oder 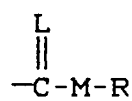 (d)

steht,

in welchen

| | |
|---|---|
| L und M | jeweils für Sauerstoff oder Schwefel stehen, |
| $R^1$, $R^2$ und $R^3$ | unabhängig voneinander für gegebenenfalls durch Halogen substituiertes $C_1$-$C_8$-Alkyl, $C_1$-$C_8$-Alkoxy, $C_1$-$C_8$-Alkylamino, Di-($C_1$-$C_8$)-Alkylamino, $C_1$-$C_8$-Alkylthio, $C_2$-$C_5$-Alkenylthio, $C_2$-$C_5$-Alkinylthio, $C_3$-$C_7$-Cycloalkylthio, für gegebenenfalls durch Halogen, Nitro, |

Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl, $C_1$-$C_4$-Halogenalkyl substituiertes Phenyl, Benzyl, Phenoxy oder Phenylthio stehen,

$R^4$ und $R^5$ unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_{20}$-Halogenalkyl, $C_1$-$C_{20}$-Alkyl oder $C_1$-$C_{20}$-Alkoxy substituiertes Phenyl, für gegebenenfalls durch Halogen, $C_1$-$C_{20}$-Alkyl, $C_1$-$C_{20}$-Halogenalkyl oder $C_1$-$C_{20}$-Alkoxy substituiertes Benzyl stehen oder zusammen für einen gegebenenfalls durch Sauerstoff unterbrochenen $C_2$-$C_6$-Alkylenring stehen,

$R^6$ für gegebenenfalls durch Halogen substituiertes $C_1$-$C_{20}$-Alkyl, das durch Sauerstoff unterbrochen sein kann für gegebenenfalls durch Halogen, $C_1$-$C_{20}$-Halogenalkyl, $C_1$-$C_{20}$-Alkoxy substituiertes Phenyl, für gegebenenfalls durch Halogen, $C_1$-$C_{20}$-Halogenalkyloder $C_1$-$C_{20}$-Alkoxy substituiertes Benzyl, für $C_2$-$C_8$-Alkenyl oder für $C_2$-$C_5$-Alkinyl steht,

sowie die enantiomerenreinen Formen von Verbindungen der Formel (I), mit der Maßgabe, daß L und M nicht gleichzeitig für Sauerstoff stehen.

Unter Einbeziehung der verschiedenen Bedeutungen (a), (b), (c) und (d) der Gruppe R der allgemeinen Formel (I) ergeben sich folgende hauptsächlichen Strukturen (Ia) bis (Id):

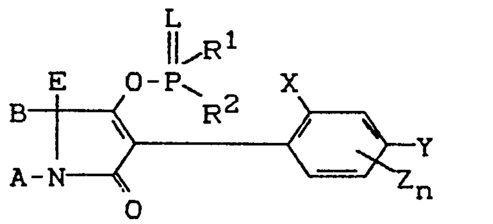

(Ia)

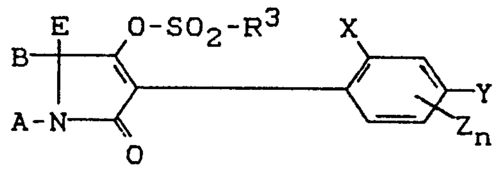

(Ib)

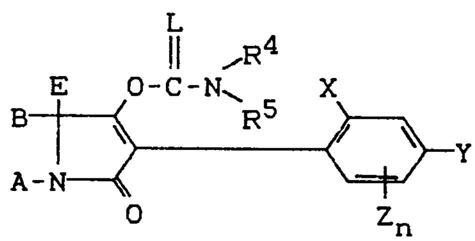

(Ic)

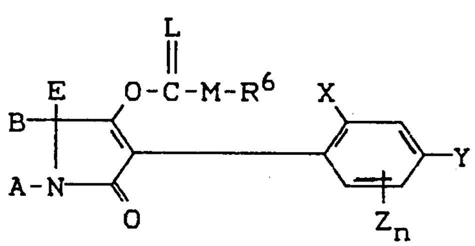

(Id)

wobei

A, B, E, L, M, X, Y, $Z_n$, $R^1$, $R^2$, $R^3$, $R^4$, $R^5$ und $R^6$

die oben angegebenen Bedeutungen besitzen.

Weiterhin wurde gefunden, daß man 3-Aryl-pyrrolidin-2,4-dione der Formel (Ia)

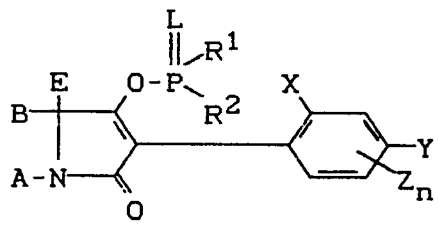

(Ia)

in welcher
A, B, E, L, X, Y, Z, $R^1$, $R^2$ und n die oben angegebene Bedeutung haben,
erhält, wenn man

A) 3-Aryl-pyrrolidin-2,4-dione der Formel (II) bzw. deren Enole

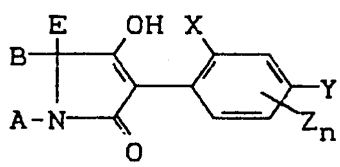

(II)

in welcher
A, B, E, X, Y, Z und n die oben angegebene Bedeutung haben
mit Phosphorverbindungen der allgemeinen Formel (III)

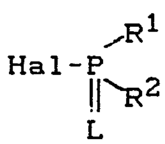

(III)

in welcher
L, $R^1$ und $R^2$ die oben angegebene Bedeutung haben
und
Hal für Halogen, insbesondere Chlor und Brom steht,
gegebenenfalls in Gegenwart eines Verdünnungsmittels, gegebenenfalls in Gegenwart eines Säurebindemittels und gegebenenfalls in Gegenwart eines Phasentransferkatalysators umsetzt.

B) Außerdem wurde gefunden, daß man Verbindungen der Formel (Ib)

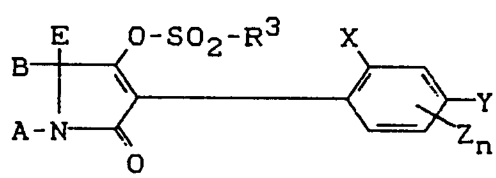

(Ib)

in welcher
A, B, E, X, Y, Z, $R^3$ und n die oben angegebene Bedeutung haben,
erhält, wenn man Verbindungen der Formel (II)

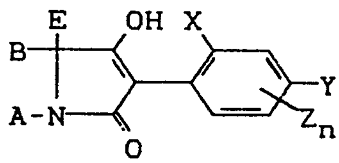 (II)

in welcher
A, B, E, X, Y, Z und n die oben angegebene Bedeutung haben
mit Sulfonsäurechloriden der allgemeinen Formel (IV)

$R^3$-$SO_2$-Cl (IV)

in welcher
$R^3$ die oben angegebene Bedeutung hat
gegebenenfalls in Gegenwart eines Verdünnungsmittels und gegebenenfalls in Gegenwart eines Säurebindemittels,
umsetzt.

C) Ferner wurde gefunden, daß man Verbindungen der Formel (Ic)

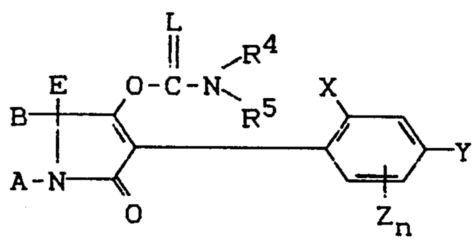 (Ic)

in welcher
A, B, E, L, X, Y, Z, $R^4$, $R^5$ und n die oben angegebene Bedeutung haben,
erhält, wenn man Verbindungen der Formel (II),

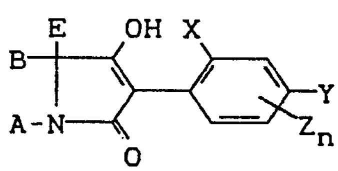 (II)

in welcher
A, B, E, X, Y, Z und n die oben angegebene Bedeutung haben
α) mit Isocyanaten der allgemeinen Formel (V)

$R^4$-N = C = O (V)

in welcher
$R^4$ die oben anGegebene Bedeutung hat
gegebenenfalls in Gegenwart eines Verdünnungsmittels und gegebenenfalls in Gegenwart eines Katalysators

β) mit Carbamidsäurechloriden oder Thiocarbamidsäurechloriden der allgemeinen Formel (VI)

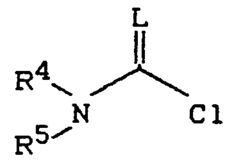

(VI)

in welcher

L, $R^4$ und $R^5$ die oben angegebene Bedeutung haben

gegebenenfalls in Gegenwart eines Verdünnungsmittels und gegebenenfalls in Gegenwart eines Säurebindemittels,

umsetzt.

D) Ferner wurde gefunden, daß man Verbindungen der Formel (Id)

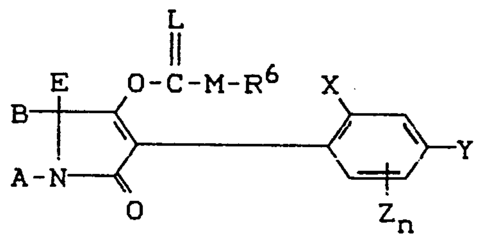

(Id)

in welcher

A, B, E, L, M, $R^6$, X, Y, Z und n die oben angegebene Bedeutung haben,

erhält, wenn man Verbindungen der Formel (II)

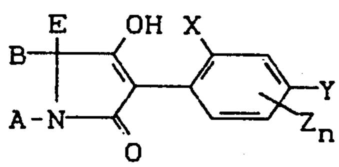

(II)

in welcher

A, B, E, X, Y, Z und n die oben angegebene Bedeutung haben

α) mit Chlormonothioameisensäureestern, Chlorameisensäurethioestern oder Chlordithioameisensäureestern der allgemeinen Formel VII

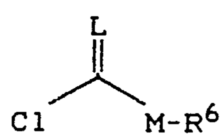

(VII)

in welcher

L, M, $R^6$ die oben angegebene Bedeutung haben

gegebenenfalls in Gegenwart eines Verdünnungsmittels und gegebenenfalls in Gegenwart eines Säurebindemittels umsetzt,

oder

β) mit Schwefelkohlenstoff und anschließend mit Alkylhalogeniden der allgemeinen Formel VIII

$R^6$-Hal (VIII)

in welcher

$R^6$ die oben angegebene Bedeutung hat

und

Hal für Chlor, Brom, Jod

steht,

umsetzt.

E) Ferner wurde gefunden, daß man Verbindungen der Formel (Ie)

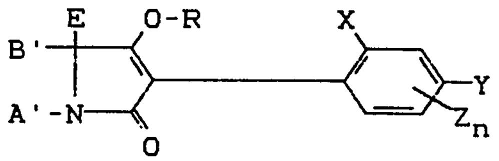

(Ie)

in welcher

E, R, X, Y, Z und n die oben angegebene Bedeutung haben,

E für Wasserstoff steht

und

A', B' zusammen für $-CH_2-CH_2-SO-CH_2-$,$-CH_2-SO-CH_2-CH_2-$ oder $CH_2-SO-CH_2-$ stehen,

erhält,

wenn man Verbindungen der Formel (If)

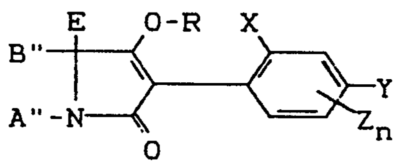

(If)

in welcher

E, R, X, Y, Z und n die oben angegebene Bedeutung haben

E für Wasserstoff steht

und

A'', B'' zusammen für $-CH_2-CH_2-S-CH_2-$,$-CH_2-S-CH_2-CH_2-$ und $-CH_2-S-CH_2-$ stehen,

mit annähernd äquimolaren Mengen eines Oxidationsmittels gegebenenfalls in Gegenwart eines Verdünnungsmittels umsetzt.

F) Ferner wurde gefunden, daß man Verbindungen der Formel (Ig)

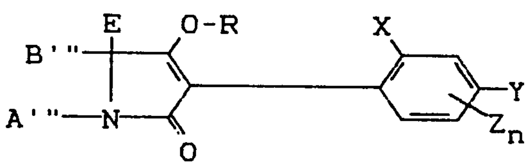

(Ig)

in welcher

E, R, X, Y, Z und n die oben angegebene Bedeutung haben,

E für Wasserstoff steht

und

A''', B''' zusammen für $-CH_2-CH_2-SO_2-CH_2-$, $-CH_2-SO_2-CH_2-CH_2-$ oder $-CH_2-SO_2-CH_2-$stehen,

erhält,

wenn man Verbindungen der Formel (If)

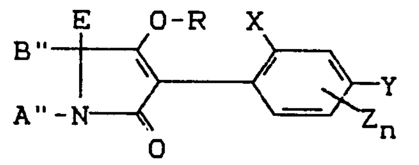 (If)

in welcher
E, R, X, Y, Z und n die oben angegebene Bedeutung haben
E für Wasserstoff steht
und
A", B" zusammen für $-CH_2-CH_2-S-CH_2-$,$-CH_2-S-CH_2-CH_2-$ und $-CH_2-S-CH_2-$ stehen,
mit mindestens doppelt äquimolaren Mengen eines Oxidationsmittels gegebenenfalls in Gegenwart eines Verdünnungsmittels umsetzt.

Überraschenderweise wurde gefunden, daß die neuen 3-Arylpyrrolidin-2,4-dion-Derivate der Formel (I) sich durch hervorragende insektizide, akarizide und herbizide Wirkungen auszeichnen.

Bevorzugt sind 3-Aryl-pyrrolidin-2,4-dion-Derivate der Formel (I), in welcher

A für gegebenenfalls durch Halogen substituiertes geradkettiges oder verzweigtes $C_1$-$C_{10}$-Alkyl, $C_3$-$C_6$-Alkenyl, $C_3$-$C_6$-Alkinyl, $C_1$-$C_8$-Alkoxy-$C_2$-$C_8$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3-7 Ringatomen, das durch 1-2 Sauerstoff- und/oder Schwefelatomen unterbrochen sein kann oder gegebenenfalls durch Halogen, $C_1$-$C_4$-Alkyl, $C_1$-$C_4$-Haloalkyl, $C_1$-$C_4$-Alkoxy, Nitro substituiertes Aryl-$C_1$-$C_4$-alkyl steht,

B und E unabhängig voneinander für Wasserstoff, geradkettiges oder verzweigtes $C_1$-$C_{10}$-Alkyl, $C_1$-$C_6$-Alkoxyalkyl stehen

oder

A und B mit dem Stickstoff- bzw. Kohlenstoffatom, an das sie gebunden sind, einen 4- bis 7-gliedrigen Zyklus bilden, der durch ein Schwefelatom, oder durch eine Sulfoxid- bzw. Sulfonylgruppe unterbrochen sein kann,

X für $C_1$-$C_4$-Alkyl, Halogen oder $C_1$-$C_4$-Alkoxy steht,

Y für Wasserstoff, $C_1$-$C_6$-Alkyl, Halogen, $C_1$-$C_4$-Alkoxy, $C_1$-$C_2$-Halogenalkyl steht,

Z für $C_1$-$C_4$-Alkyl, Halogen, $C_1$-$C_4$-Alkoxy steht,

n für eine Zahl von 0 - 3 steht,

R für die Gruppen

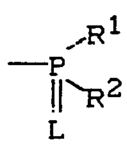 (a) $-SO_2-R^3$ (b)

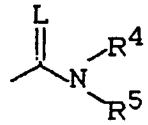 (c) 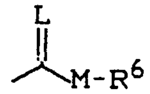 (d)

steht,
in welchen

L und M jeweils für Sauerstoff oder Schwefel stehen,

$R^1$, $R^2$ und $R^3$ unabhängig voneinander für gegebenenfalls durch Halogen substituiertes $C_1$-$C_6$-Alkyl, $C_1$-$C_6$-Alkoxy, $C_1$-$C_6$-Alkylamino, Di-($C_1$-$C_6$)-Alkylamino, $C_1$-$C_6$-Alkylthio, $C_3$-$C_4$-Alkenylthio, $C_2$-$C_4$-Alkinylthio, $C_3$-$C_6$-Cycloalkylthio, für gegebenenfalls durch Fluor, Chlor, Brom, Nitro, Cyano, $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkyl, $C_1$-$C_3$-Halogenalkyl substituiertes Phenyl, Benzyl, Phenoxy oder Phenylthio stehen,

$R^4$ und $R^5$ unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_5$-Halogenalkyl, $C_1$-$C_5$-Alkyl oder $C_1$-$C_5$-Alkoxy substituiertes Phenyl, für

gegebenenfalls durch Halogen, $C_1$-$C_5$-Alkyl,$C_1$-$C_5$-Halogenalkyl oder $C_1$-$C_5$-Alkoxy substituiertes Benzyl stehen,

$R^6$ für gegebenenfalls durch Halogen substituiertes $C_1$-$C_{20}$-Alkyl, das durch Sauerstoff unterbrochen sein kann für gegebenenfalls durch Halogen, $C_1$-$C_5$-Halogenalkyl, $C_1$-$C_5$-Alkoxy substituiertes Phenyl, für gegebenenfalls durch Halogen, $C_1$-$C_5$-Halogenalkyl oder $C_1$-$C_5$-Alkoxy substituiertes Benzyl steht,

sowie die enantiomerenreinen Formen von Verbindungen der Formel (I), mit der Maßgabe, daß L und M nicht gleichzeitig für Sauerstoff stehen.

Besonders bevorzugt sind Verbindungen der Formel (I)

in welcher

A für gegebenenfalls durch Halogen substituiertes geradkettiges oder verzweigtes $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3-6 Ringatomen, das durch 1-2 Sauerstoff- und/oder Schwefelatome unterbrochen sein kann oder für gegebenenfalls durch Fluor, Chlor, Brom, Methyl, Ethyl, Propyl, isoPropyl, Methoxy, Ethoxy, Trifluormethyl, Nitro substituiertes Aryl-$C_1$-$C_3$-alkyl steht,

B, E unabhängig voneinander für Wasserstoff, geradkettiges oder verzweigtes $C_1$-$C_8$-Alkyl, $C_1$-$C_4$-Alkoxyalkyl stehen, oder

A und B mit dem Stickstoff- bzw. Kohlenstoffatom an das sie gebunden sind, einen 5-6-gliedrigen Zyklus bilden, der durch ein Schwefelatom oder durch eine Sulfoxid- bzw. Sulfonylgruppe unterbrochen sein kann,

X für Methyl, Ethyl, Propyl, i-Propyl, Fluor, Chlor, Brom, Methoxy oder Ethoxy steht,

Y für Wasserstoff, Methyl, Ethyl, Propyl, i-Propyl, Butyl, i-Butyl, tert.-Butyl, Fluor, Chlor, Brom, Methoxy, Ethoxy oder Trifluormethyl steht,

Z für Methyl, Ethyl, i-Propyl, Butyl, i-Butyl, tert.-Butyl, Fluor, Chlor, Brom, Methoxy oder Ethoxy steht,

n für eine Zahl von 0-3 steht,

R für die Gruppen

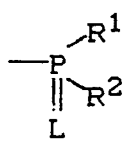 (a) $-SO_2-R^3$ (b)

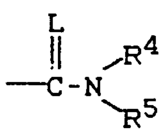 (c) oder 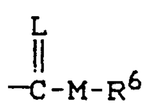 (d)

steht,

in welchen

L und M jeweils für Sauerstoff oder Schwefel stehten,

$R^1$, $R^2$ und $R^3$ unabhängig voneinander für gegebenenfalls durch Fluor oder Chlor substituiertes $C_1$-$C_4$-Alkyl, $C_1$-$C_4$-Alkoxy, $C_1$-$C_4$-Alkylamino, Di-($C_1$-$C_4$-Alkyl)amino, $C_1$-$C_4$-Alkylthio, für gegebenenfalls durch Fluor, Chlor, Brom, Nitro, Cyano, $C_1$-$C_2$-Alkoxy, $C_1$-$C_4$-Fluoralkoxy, $C_1$-$C_2$-Chloralkoxy, $C_1$-$C_2$-Alkylthio, $C_1$-$C_2$-Fluoralkylthio, $C_1$-$C_2$-Chloralkylthio, $C_1$-$C_3$-Alkyl substituiertes Phenyl, Benzyl, Phenoxy oder Phenylthio stehen,

$R^4$ und $R^5$ unabhängig voneinander für gegebenenfalls durch Fluor, Chlor, Brom substituiertes $C_1$-$C_{10}$-Alkyl, $C_1$-$C_{10}$-Alkoxy, $C_1$-$C_{10}$-Alkoxy-($C_1$-$C_{10}$)alkyl, für gegebenenfalls durch Fluor, Chlor, Brom, $C_1$-$C_{20}$-Halogenalkyl, $C_1$-$C_{20}$-Alkyl oder $C_1$-$C_4$-Alkoxy substituiertes Phenyl, für gegebenenfalls durch Fluor, Chlor, Brom, $C_1$-$C_4$-Alkyl, $C_1$-$C_4$-Halogenalkyl oder $C_1$-$C_4$-Alkoxy substituiertes Benzyl stehen,

$R^6$ für gegebenenfalls durch Fluor, Chlor, Brom substituiertes $C_1$-$C_{10}$-Alkyl, das durch Sauerstoff unterbrochen sein kann für gegebenenfalls durch Fluor, Chlor, Brom, $C_1$-$C_4$-Halogenalkyl, $C_1$-$C_4$-Alkoxy substituiertes Phenyl, für gegebenenfalls durch Fluor,

Chlor, Brom, $C_1$-$C_4$-Halogenalkyl oder $C_1$-$C_4$-Alkoxy substituiertes Benzyl steht,
sowie die enantiomerenreinen Formen von Verbindungen der Formel (I), mit der Maßgabe, daß L und M nicht gleichzeitig für Sauerstoff stehen.

Verwendet man gemäß Verfahren (A) 3-(2,4,6-Trimethylphenyl)-1,5-trimethylen-2,4-pyrrolidin-dion und Methanthio-phosphonsäurechlorid-(2,2,2-trifluorethylester) als Ausgangsprodukte, so kann der Reaktionsverlauf durch folgendes Reaktionsschema wiedergegeben werden:

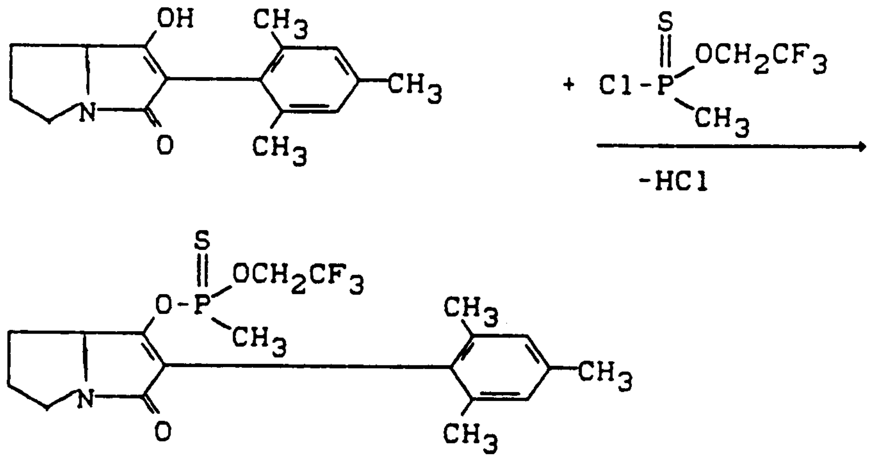


Verwendet man gemäß Verfahren (B) 3-(2,4,6-Trimethyl phenyl)-1,5-trimethylen-2,4-pyrrolidin-dion und Methansulfonsäurechlorid als Ausgangsprodukte, so kann der Reaktionsverlauf durch folgendes Reaktionsschema wiedergegeben werden:

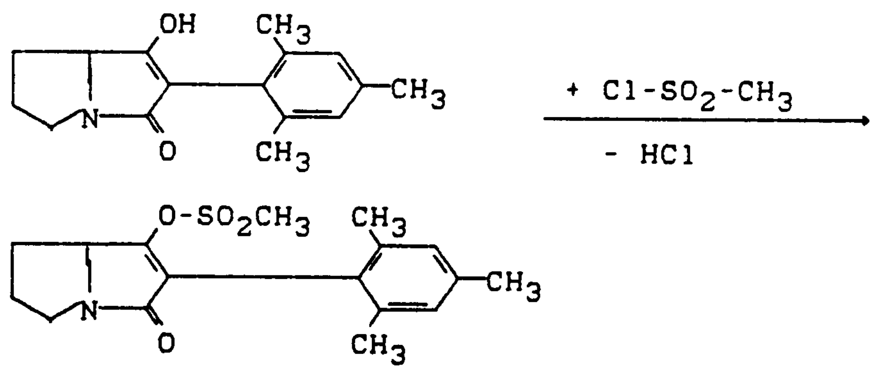


Verwendet man gemäß Verfahren ($C_\alpha$) 3-(2,4,6-Trimethyl phenyl)-1,5-trimethylen-2,4-pyrrolidin-dion und Ethylisocyanat als Ausgangsprodukte, so kann der Reaktionsverlauf durch folgendes Schema wiedergegeben werden:

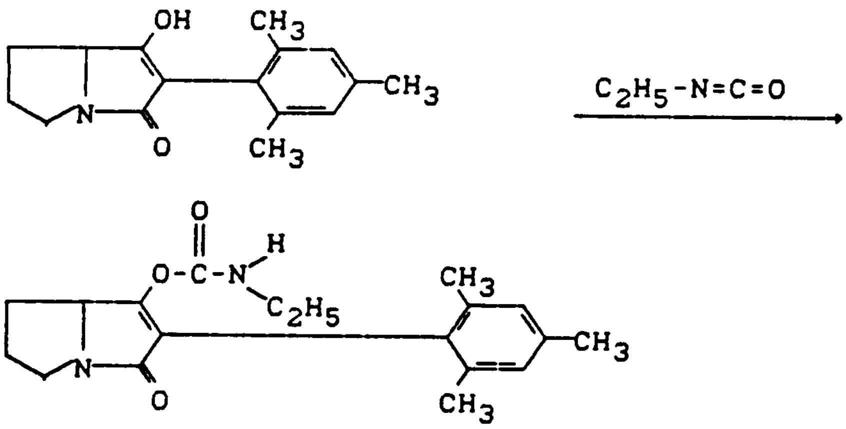


Verwendet man gemäß Verfahren ($C_\beta$) 3-(2,4,6-Trimethylphenyl)-1,5-trimethylen-2,4-pyrrolidin-dion und Dimethylcarbamidsäurechlorid als Ausgangsprodukte, so kann der Reaktionsverlauf wie folgt wiedergegeben werden:

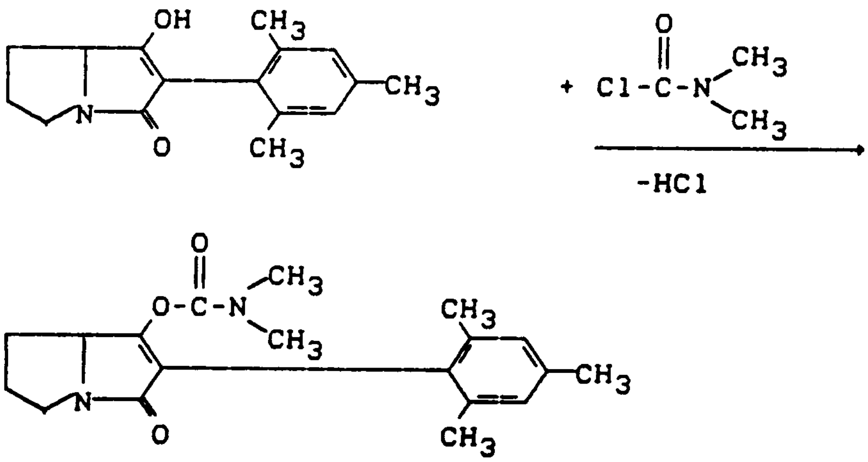


Verwendet man gemäß Verfahren ($D_\alpha$) 3-(2,4,6-Trimethylphenyl)-1,5-tetramethylen-2,4-pyrrolidin-dion und Chlormonothioameisensäuremethylester als Ausgangsprodukte, so kann der Reaktionsverlauf wie folgt wiedergegeben werden:

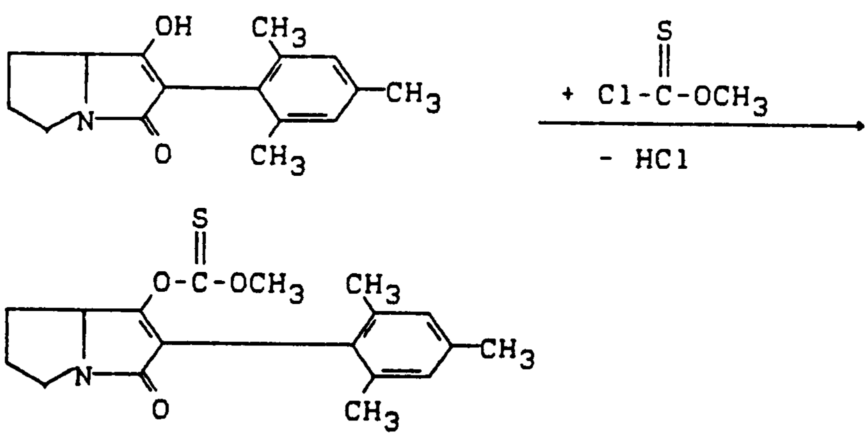


Verwendet man gemäß Verfahren ($D_\beta$) 3-(2,4,6-Trimethylphenyl)-1,5-tetramethylen-2,4-pyrrolidin-dion, Schwefelkohlenstoff und Methyliodid als Ausgangskomponenten, so kann der Reaktionsverlauf wie folgt wiedergegeben werden:

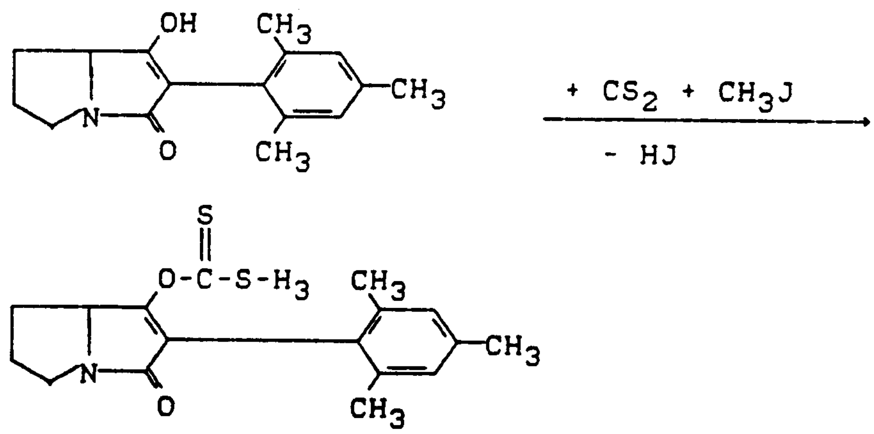


Verfahren (E) kann wie folgt wiedergegeben werden:

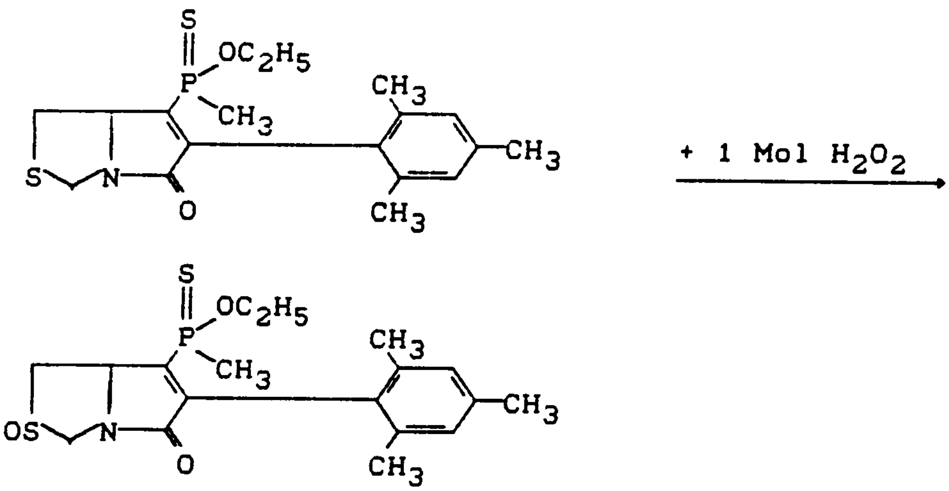


Verfahren (F) kann wie folgt wiedergegeben werden:

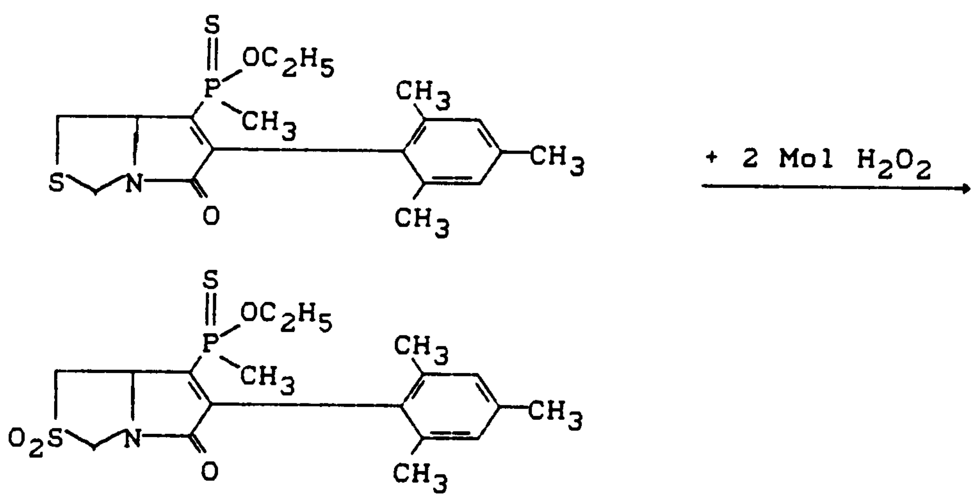

Die bei den obigen Verfahren (A)-(D) als Ausgangsstoffe benötigten Verbindungen der Formel (II)

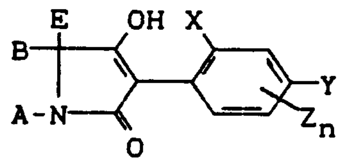

(II)

in welcher
A, B, E, X, Y, Z und n die oben angegebene Bedeutung haben, sind neu, aber Gegenstand früherer eigener Anmeldungen. So erhält man Verbindungen der Formel (II) wenn man
N-Acylaminosäureester der Formel (IX)

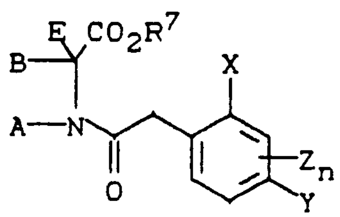

(IX)

in welcher A, B, E, X, Y, Z und n die oben angegebene Bedeutung haben und
$R^7$ für Alkyl steht,
in Gegenwart eines Verdünnungsmittels und in Gegenwart einer Base intramolekular kondensiert.

Die bei dem obigen Verfahren als Ausgangsstoffe benötigten Verbindungen der Formel (IX)

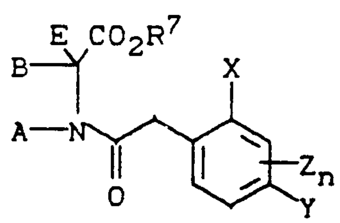

(IX)

in welcher
A, B, E, X, Y, Z, n und $R^7$ die oben angegebene Bedeutung haben sind teilweise bekannt oder lassen sich nach im Prinzip bekannten Methoden in einfacher Weise herstellen. So erhält man z.B. Acyl-aminosäureester der Formel (II), wenn man

a) Aminosäureester der Formel (X),

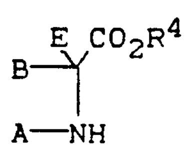

(X)

in welcher
$R^4$ für Wasserstoff (Xa) und Alkyl (Xb) steht
und
A, B und E die oben angegebene Bedeutung haben mit Phenylessigsäurehalogeniden der Formel (XI)

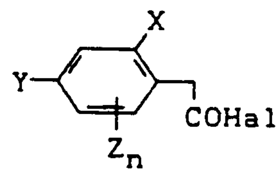

(XI)

in welcher
X, Y, Z und n die oben angegebene Bedeutung haben
und
Hal für Chlor oder Brom steht,
acyliert (Chem. Reviews 52 237-416 (1953));
oder wenn man Acylaminosäuren der Formel (IIa),

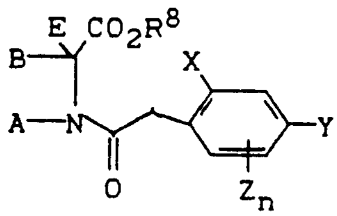

(IIa)

in welcher
A, B, E, X, Y, Z und n die oben angegebene Bedeutung haben
und
$R^8$ für Wasserstoff steht,
verestert (Chem. Ind. (London) 1568 (1968).

Beim Herstellungsverfahren A) setzt man zum Erhalt von Verbindungen der Struktur (Ia) auf 1 Mol der Verbindung (II), 1 bis 2, vorzugsweise 1 bis 1,3 Mol der Phosphorverbindung der Formel (III) bei Temperaturen zwischen -40 und 150 °C, vorzugsweise zwischen -10 und 110 °C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Beim Herstellungsverfahren B) setzt man pro Mol Ausgangsverbindung der Formel (II) ca. 1 Mol Sulfonsäurechlorid (IV) bei 0 bis 150 °C, vorzugsweise bei 20 bis 70 °C um.

Als gegebenenfalls zugesetzte Verdünnungsmittel kommen alle inerten, polaren organischen Lösungsmittel in Frage wie Ether, Amide, Nitrile, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung II dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren B kann gegebenenfalls unter Phasen-Transfer-Bedingungen gearbeitet werden (W.J. Spillane et al., J. Chem. Soc. Perkin Trans I, (3) 677-9 (1982)). In diesem Fall setzt man pro Mol Ausgangsverbindung der Formel (II) 0,3 bis 1,5 Mol Sulfonsäurechlorid (IV), bevorzugt 0,5 Mol bei 0 bis

150 °C, vorzugsweise bei 20 bis 70 °C um. Als Phasen-Transfer-Katalysatoren können z.B. alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren $C_\alpha$ setzt man pro Mol Ausgangsverbindung der Formel II ca. 1 Mol Isocyanat der Formel (V) bei 0 bis 100 °C, vorzugsweise bei 20 bis 50 °C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren $C_\beta$ setzt man pro Mol Ausgangsverbindung der Formel (II) ca. 1 Mol Carbamidsäurechlorid bzw. Thiocarbamidsäurechlorid der Formel (VI) bei 0 bis 150 °C, vorzugsweise bei 20 bis 70 °C um.

Als gegebenenfalls zugesetzte Verdünnungsmittel kommen alle inerten polaren organischen Lösungsmittel in Frage wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung II dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren $D_\alpha$ setzt man pro Mol Ausgangsverbindung der Formel (II) ca. 1 Mol Chlormonothioameisensäureester bzw. Chlordithioameisensäureester der Formel (VII) bei 0 bis 120 °C, vorzugsweise bei 20 bis 60 °C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung II dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren $D_\beta$ setzt man pro Mol Ausgangsverbindung der Formel (II) die äquimolare Menge bzw. einen Überschuß Schwefelkohlenstoff zu. Man arbeitet hierbei vorzugsweise bei Temperaturen von 0 bis 50 °C und insbesondere bei 20 bis 30 °C.

Oft ist es zweckmäßig zunächst aus der Verbindung der Formel (II) durch Zusatz eines Deprotonierungsmittels (wie z.B. Kaliumtertiärbutylat oder Natriumhydrid) das entsprechende Salz herzustellen, Man setzt die Verbindung (II) solange mit Schwefelkohlenstoff um bis die Bildung der Zwischenverbindung abgeschlossen ist, z.B. mehrstündiges Rühren bei Raumtemperatur.

Die weitere Umsetzung mit dem Alkylhalogenid der Formel (VIII) erfolgt vorzugsweise bei 0 bis 70 °C und insbesondere bei 20 bis 50 °C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Bei der Durchführung des erfindungsgemäßen Verfahrens (E) werden die Ausgangsstoffe der Formel (If) und das entsprechende Oxidationsmittel in angenähert äquimolaren Mengen eingesetzt. Die Aufarbeitung erfolgt nach den üblichen Methoden.

Als Oxidationsmittel kommen für das erfindungsgemäße Verfahren (E) alle schwefeloxidierenden Reagenzien in Frage, z.B. Halogen wie Chlor und Brom und deren wäßrige Lösungen, Alkaliperoxide wie Natriumperoxid und Kaliumperoxid, Salze von Halogensauerstoffsäuren wie Kaliumchlorat, Kaliumbromat, Natriumperjodat und Natriumperborat, weiterhin anorganische Persalze wie Kaliumpermanganat, Kaliumperoxodisulfat und Kaliumperoxomonosulfat, aber auch $H_2O_2$ in Gegenwart von Übergangsmetallsalzen wie Natriumwolframat und Ammoniummolybdat. Weiterhin verwendbar sind organische Peroxide wie tert.-

Butylhydroperoxid aber auch organische Persäuren wie Peressigsäure, Perpropionsäure und m-Chlorperbenzoesäure (MCPBA).

Als Verdünnungsmittel können bei dem erfindungsgemäßen Verfahren (E) alle gegenüber diesen Verbindungen inerten Solventien eingesetzt werden. Vorzugsweise verwendbar sind Kohlenwasserstoffe wie Benzin, Benzol, Toluol, Xylol und Tetralin, ferner Halogenkohlenwasserstoffe wie Methylenchlorid, Chloroform, Chlorbenzol und Dichlorbenzol, außerdem organische Säuren wie Essigsäure und Propionsäure und Wasser.

Arbeitet man in Gegenwart eines Verdünnungsmittels, so können die Reaktionstemperaturen bei der Durchführung des erfindungsgemäßen Verfahrens (E) innerhalb eines größeren Bereiches variiert werden. Im allgemeinen liegen die Reaktionstemperaturen zwischen -30 °C und +150 °C, vorzugsweise zwischen 0 °C und 100 °C.

Das erfindungsgemäße Verfahren (E) wird im allgemeinen unter Normaldruck durchgeführt.

Bei der Durchführung des erfindungsgemäßen Verfahrens (F) werden die Ausgangsstoffe der Formel (If) und das entsprechende Oxidationsmittel in angenähert doppelt äquimolaren Mengen eingesetzt. Es ist jedoch auch möglich, das Oxidationsmittel in einem größeren Überschuß (bis zu 2 Mol) einzusetzen. Die Aufarbeitung erfolgt nach den üblichen Methoden.

Als Oxidationsmittel kommen für das erfindungsgemäße Verfahren (F) alle schwefeloxidierenden Reagenzien in Frage, z.B. Halogen wie Chlor und Brom und deren wäßrige Lösungen, Alkaliperoxide wie Natriumperoxid und Kalium-peroxid, Salze von Halogensauerstoffsäuren wie Kaliumchlorat, Kaliumbromat, Natriumperjodat und Natriumperborat, weiterhin anorganische Persalze wie Kaliumpermanganat, Kaliumperoxodisulfat und Kaliumperoxomonosulfat, aber auch $H_2O_2$ in Gegenwart von Übergangsmetallsalzen wie Natriumwolframat und Ammoniummolybdat. Weiterhin verwendbar sind organische Peroxide wie tert.-Butylhydroperoxid aber auch organische Persäuren wie Peressigsäure, Perpropionsäure und m-Chlorperbenzoesäure (MCPBA).

Als Verdünnungsmittel können bei dem erfindungsgemäßen Verfahren (F) alle gegenüber diesen Verbindungen inerten Solventien eingesetzt werden. Vorzugsweise verwendbar sind Kohlenwasserstoffe wie Benzin, Benzol, Toluol, Xylol und Tetralin, ferner Halogenkohlenwasserstoffe wie Methylenchlorid, Chloroform, Chlorbenzol und Dichlorbenzol, außerdem organische Säuren wie Essigsäure und Propionsäure und Wasser.

Arbeitet man in Gegenwart eines Verdünnungsmittels, so können die Reaktionstemperaturen bei der Durchführung des erfindungsgemäßen Verfahrens (F) innerhalb eines größeren Bereiches variiert werden. Im allgemeinen liegen die Reaktionstemperaturen zwischen -30 °C und +150 °C, vorzugsweise zwischen 0 °C und 100 °C.

Das erfindungsgemäße Verfahren (F) wird im allgemeinen unter Normaldruck durchgeführt.

Beispiel 1

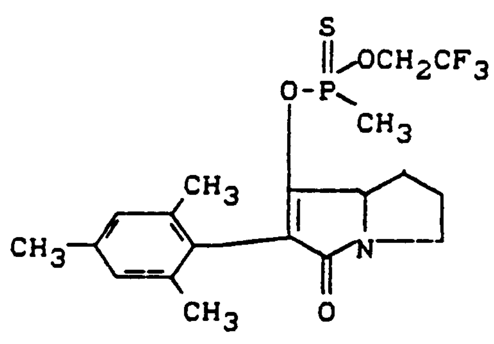

11,1 g 3-(2,4,6-Trimethyl-phenyl)-1,5-trimethylen-2,4-pyrrolidin-dion(0,04 Mol), 7 g (0,05 Mol) Kaliumcarbonat und 0,5 g (DABCO) (1,4-Diazabicyclo[2.2.2]octan) (4,5 mmol) werden in 100 ml Acetonitril suspendiert und bei 20 °C mit 9,5 g (0,05 Mol) Methanthiophosphonsäurechlorid-(2,2,2-trfluorethylester) versetzt und einen Tag bei 20 °C gerührt. Anschließend wird der Feststoff abgesaugt und die Lösung einrotiert. Der Rückstand wird über Kieselgel filtriert (Laufmitel: Hexan: Essigsäureethylester = 8:2).

Man erhält 8,9 g (52 % der Theorie)
4-(2,2,2-Trifluorethoxy-methanthiophosphoryl)-3-(2,4,6-trimethylphenyl)-1,5-trimethylen-3-pyrrolin-2-on vom

Schmelzpunkt 109 ° C.

Analog werden erhalten:

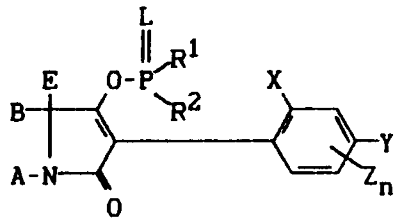

(Ia)

| Beispiel-Nr. | X | Y | Zn | L | $R^1$ | $R_2$ | A B | E | physik. Daten |
|---|---|---|---|---|---|---|---|---|---|
| 2 | $CH_3$ | $CH_3$ | 6-$CH_3$ | S | $OC_2H_5$ | $nC_3H_7S$ | $-(CH_2)_3-$ | H | $n_D^{20}$ 1,5662 |
| 3 | $CH_3$ | $CH_3$ | 6-$CH_3$ | O | $N(CH_3)_2$ | $N(CH_3)_2$ | $-(CH_2)_3-$ | H | $n_D^{20}$ 1,5382 |
| 4 | $CH_3$ | $CH_3$ | 6-$CH_3$ | S | $CH_3$ | $SCH_2-C(CH_3)_3$ | $-(CH_2)_3-$ | H | $n_D^{20}$ 1,5636 |
| 5 | $CH_3$ | $CH_3$ | 6-$CH_3$ | S | $CH_3$ | $OCH_3$ | $-(CH_2)_3-$ | H | $n_D^{20}$ 1,5344 |
| 6 | $CH_3$ | $CH_3$ | 6-$CH_3$ | S | $CH_3$ | $OC_2H_5$ | $-(CH_2)_3-$ | H | $n_D^{20}$ 1,5507 |
| 7 | $CH_3$ | $CH_3$ | 6-$CH_3$ | S | $CH_3$ | $OC_3H_7$-i | $-(CH_2)_3-$ | H | Fp: 32° C |
| 8 | $CH_3$ | $CH_3$ | 6-$CH_3$ | S | $CH_3$ | $OC_4H_9$-sec | $-(CH_2)_3-$ | H | Fp: 89° C |
| 9 | $CH_3$ | $CH_3$ | 6-$CH_3$ | S | $CH_3$ | $OC_4H_9$-iso | $-(CH_2)_3-$ | H | |
| 10 | $CH_3$ | $CH_3$ | 6-$CH_3$ | S | $C_2H_5$ | $OCH_3$ | $-(CH_2)_3-$ | H | Fp: 40° C |
| 11 | $CH_3$ | $CH_3$ | 6-$CH_3$ | S | $C_2H_5$ | $OC_3H_7$-i | $-(CH_2)_3-$ | H | |
| 12 | $CH_3$ | $CH_3$ | 6-$CH_3$ | S | $C_2H_5$ | $OC_4H_9$-sec | $-(CH_2)_3-$ | H | |
| 13 | $CH_3$ | $CH_3$ | 6-$CH_3$ | S | $C_2H_5$ | $OC_4H_9$-i | $-(CH_2)_3-$ | H | |

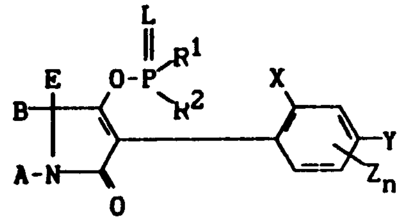

(Ia)

(Fortsetzung)

| Beispiel-Nr. | X | Y | Zn | L | $R^1$ | $R_2$ | A B | E | physik. Daten |
|---|---|---|---|---|---|---|---|---|---|
| 14 | $CH_3$ | $CH_3$ | $6\text{-}CH_3$ | S | $C_2H_5$ | $SCH_3$ | $-(CH_2)_4-$ | H | $n_D^{20}$ 1,5662 |
| 15 | $CH_3$ | $CH_3$ | $6\text{-}CH_3$ | S | $C_2H_5$ | $SC_2H_5$ | $-(CH_2)_4-$ | H | |
| 16 | $CH_3$ | $CH_3$ | $6\text{-}CH_3$ | S | $C_2H_5$ | $SC_3H_7\text{-}i$ | $-(CH_2)_4-$ | H | |
| 17 | $CH_3$ | $CH_3$ | $6\text{-}CH_3$ | S | $C_2H_5$ | $SCH_2CH{=}CH_2$ | $-(CH_2)_4-$ | H | |
| 18 | $CH_3$ | $CH_3$ | $6\text{-}CH_3$ | S | $C_2H_5$ | $SCH_2C{\equiv}CH$ | $-(CH_2)_4-$ | H | |
| 19 | $CH_3$ | $CH_3$ | $6\text{-}CH_3$ | S | $C_2H_5$ | $SC_4H_9\text{-sec.}$ | $-(CH_2)_4-$ | H | |
| 20 | $CH_3$ | $CH_3$ | $6\text{-}CH_3$ | S | $C_2H_5$ | $SC_4H_9\text{-iso}$ | $-(CH_2)_4-$ | H | |
| 21 | $CH_3$ | $CH_3$ | $6\text{-}CH_3$ | S | $C_2H_5$ | 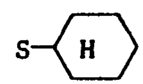 | $-(CH_2)_4-$ | H | |
| 22 | $CH_3$ | $CH_3$ | $6\text{-}CH_3$ | S | $CH_3$ | $SC_3H_7\text{-n}$ | $-(CH_2)_4-$ | H | |
| 23 | $CH_3$ | $CH_3$ | $6\text{-}CH_3$ | S | $OC_2H_5$ | $OC_2H_5$ | $-(CH_2)_4-$ | H | Fp.: 35°C |

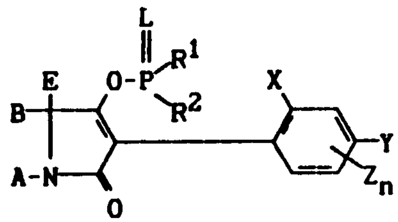

(Ia) (Fortsetzung)

| Beispiel-Nr. | X | Y | Zn | L | $R^1$ | $R_2$ | A | B | E | physik. Daten |
|---|---|---|---|---|---|---|---|---|---|---|
| 24 | $CH_3$ | $CH_3$ | 6-$CH_3$ | S | 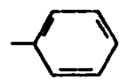 | $OC_2H5$ | $-(CH_2)_4-$ | | H | |
| 25 | $CH_3$ | $CH_3$ | 6-$CH_3$ | S | $CH_3$ | $OC_3H_7$-i | $C_3H_7$-i | H | H | |
| 26 | $CH_3$ | $CH_3$ | 6-$CH_3$ | S | $CH_3$ | $OC_3H_7$-i | 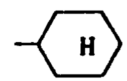 | H | H | |
| 27 | $CH_3$ | $CH_3$ | 6-$CH_3$ | S | $C_2H_5$ | 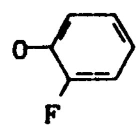 | $-(CH_2)_4-$ | | H | |
| 28 | $CH_3$ | $CH_3$ | 6-$CH_3$ | S | $OC_2H_5$ | 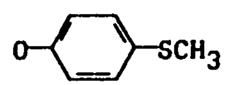 | $-(CH_2)_4-$ | | H | |
| 29 | $CH_3$ | $CH_3$ | 6-$CH_3$ | S | n-$C_4H_9$ | 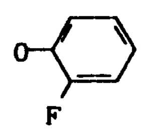 | $-(CH_2)_4-$ | | H | |

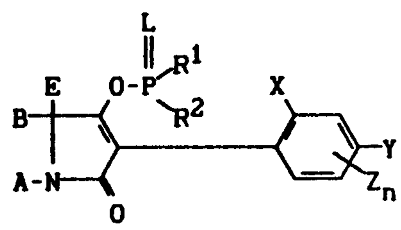

(Ia)

(Fortsetzung)

| Beispiel-Nr. | X | Y | Zn | L | $R^1$ | $R_2$ | A | B | E | physik. Daten |
|---|---|---|---|---|---|---|---|---|---|---|
| 30 | $CH_3$ | $CH_3$ | 6-$CH_3$ | S | $CH_3$ | $OC_2H_5$ | -$(CH_2)_4$- | | H | Fp. 42°C |
| 31 | $CH_3$ | $CH_3$ | 6-$CH_3$ | S | $C_2H_5$ | $OCH_3$ | -$(CH_2)_4$- | | H | $n_D^{20}$ 1,5757 |
| 32 | $CH_3$ | $CH_3$ | 6-$CH_3$ | S | $C_2H_5$ | $OC_2H_5$ | -$(CH_2)_4$- | | H | Fp. 75°C |
| 33 | $CH_3$ | $CH_3$ | 6-$CH_3$ | S | $CH_3$ | $OC_3H_7$-i | -$(CH_2)_4$- | | H | Fp. 32°C |
| 34 | $CH_3$ | $CH_3$ | 6-$CH_3$ | S | $C_2H_5$ | $OCH_3$ | 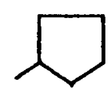 | H | H | $n_D^{20}$ 1,5515 |
| 35 | $CH_3$ | $CH_3$ | 6-$CH_3$ | S | $OC_2H_5$ | $OC_2H_5$ | $C_3H_7$-i | $CH_3$ | H | $n_D^{20}$ 1,5076 |
| 36 | $CH_3$ | $CH_3$ | 6-$CH_3$ | S | $CH_3$ | $OC_2H_5$ | 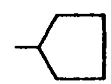 | H | H | $n_D^{20}$ 1,5505 |

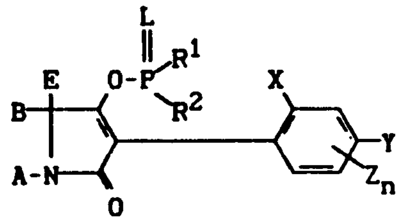

(Ia)

(Fortsetzung)

| Beispiel-Nr. | X | Y | Zn | L | $R^1$ | $R_2$ | A | B | E | physik. Daten |
|---|---|---|---|---|---|---|---|---|---|---|
| 37 | $CH_3$ | $CH_3$ | 6-$CH_3$ | S | $CH_3$ | $OC_3H_7$-i | 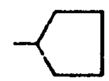 | H | H | $n_D^{20}$ 1,5452 |
| 38 | $CH_3$ | $CH_3$ | 6-$CH_3$ | S | $CH_3$ | $OC_2H_5$ | $C_3H_7$-i | $CH_3$ | H | $n_D^{20}$ 1,5194 |
| 39 | $CH_3$ | $CH_3$ | 6-$CH_3$ | S | $C_2H_5$ | $OCH_3$ | $C_3H_7$-i | $CH_3$ | H | $n_D^{20}$ 1,5328 |
| 40 | $CH_3$ | $CH_3$ | 6-$CH_3$ | S | $C_2H_5$ | $OC_2H_5$ | $C_3H_7$-i | $CH_3$ | H | $n_D^{20}$ 1,5237 |
| 41 | $CH_3$ | $CH_3$ | 6-$CH_3$ | S | $OC_2H_5$ | $OC_2H_5$ | $-(CH_2)_3-$ | | H | Fp. 42°C |
| 42 | $CH_3$ | $CH_3$ | 6-$CH_3$ | O | $OC_2H_5$ | $SC_3H_7$-i | $-(CH_2)_3-$ | | H | $n_D^{20}$ 1,5392 |
| 43 | $CH_3$ | $CH_3$ | 6-$CH_3$ | S | $OCH_3$ | $N(CH_3)_2$ | $-(CH_2)_3-$ | | H | |
| 44 | $CH_3$ | $CH_3$ | 6-$CH_3$ | S | $CH_3$ | $SCH(CH_3)C_2H_5$ | $-(CH_2)_3-$ | | H | $n_D^{20}$ 1,5412 |
| 45 | $CH_3$ | $CH_3$ | 6-$CH_3$ | O | $C_2H_5O$ | $SCH(CH_3)C_2H_5$ | $-(CH_2)_3-$ | | H | $n_D^{20}$ 1,5405 |

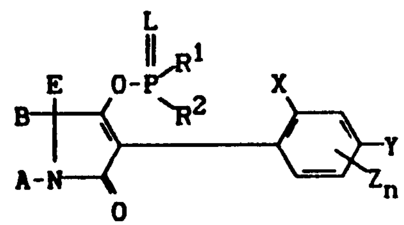

(Ia)

(Fortsetzung)

| Beispiel-Nr. | X | Y | Zn | L | $R^1$ | $R_2$ | A B | E | physik. Daten |
|---|---|---|---|---|---|---|---|---|---|
| 46 | $CH_3$ | $CH_3$ | 6-$CH_3$ | O | $C_2H_5O$ | $SC_3H_7$-i | $-(CH_2)_3-$ | H | |
| 47 | $CH_3$ | $CH_3$ | 6-$CH_3$ | O | $CH_3O$ | $SC_3H_7$-i | $-(CH_2)_3-$ | H | |
| 48 | $CH_3$ | $CH_3$ | 6-$CH_3$ | O | n-$C_4H_9O$ | $SC_3H_7$-i | $-(CH_2)_3-$ | H | |
| 49 | $CH_3$ | $CH_3$ | 6-$CH_3$ | O | $CH_3O$ | $SC_4H_9$-sek | $-(CH_2)_3-$ | H | |
| 50 | $CH_3$ | $CH_3$ | 6-$CH_3$ | O | n-$C_4H_9$ | $SC_4H_9$-sek | $-(CH_2)_3-$ | H | |

Beispiel 51

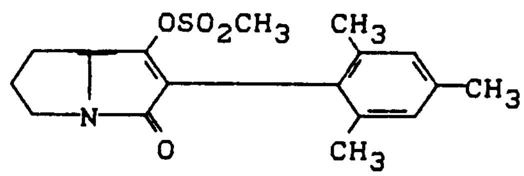

2,58 g (0,01 Mol) 3-(2,4,6-Trimethyl-phenyl)-1,5-trimethylen-2,4-pyrrolidin-dion werden in 20 ml absolutem Dimethylformamid bei Raumtemperatur suspendiert. Man versetzt portionsweise mit 0,3 g einer 80 %igen Natriumhydridsuspension in Paraffinöl. Es wird solange nachgerührt, bis die Wasserstoff-Entwicklung beendet ist. Dann wird auf 10 °C abgekühlt und mit 1,6 g (0,014 Mol) Methansulfonsäurechlorid versetzt. Man rührt 1 Stunde bei Raumtemperatur nach und rührt dann in 100 ml dest. Wasser ein, man extrahiert mit Methylenchlorid (3 mal), wäscht die organische Phase einmal mit 40 ml 5 %iger Salzsäure, trocknet die organische Phase über Natriumsulfat und zieht danach das Lösungsmittel im Rotationsverdampfer ab.

Zur Reinigung des Rohproduktes wird chromatographiert (Laufmittelgemisch: Essigester/Cyclohexan 1:1) Ausbeute: 2,51 g (75 %) der Titelverbindung

Beispiel 52

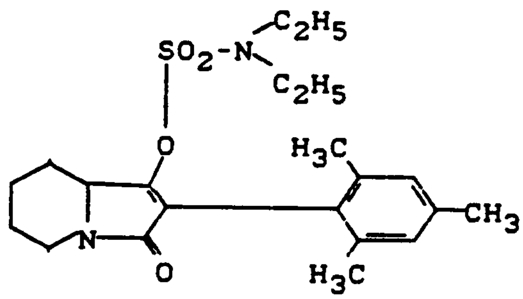

2,71 g (0,01 Mol) 3-(2,4,6-Trimethyl-phenyl)-1,5-tetramethylen-2,4-pyrrolidin-dion werden in 15 ml Toluol suspendiert. Es werden nacheinander 62 mg (0,2 mmol) tetra-Butylammoniumbromid, 0,86 g (0,005 Mol) Diethylsulfamoylchlorid und 15 ml 30 gew.-%ige Natronlauge zugegeben.

Unter kräftigem Rühren wird auf 50 °C gebracht. Bei dieser Temperatur läßt man 1,5 Stunden reagieren. Dann wird mit 100 ml Methylenchlorid und 100 ml Wasser versetzt, die organischen Phasen werden abgetrennt, die wäßrige Phase wird 2 mal mit Methylenchlorid nachextrahiert. Die vereinigten organischen Phasen werden mit Wasser gewaschen und über $Na_2SO_4$ getrocknet.

Zur Reinigung wird chromatographiert (Laufmittel: Essigester/Cyclohexan 1:3)
Ausbeute: 400 mg (10 %) farbloses, zähes Öl

Analog werden erhalten:

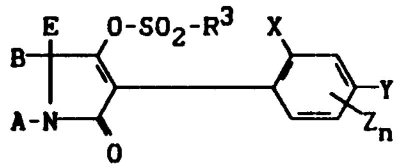

(Ib)

| Beispiel-Nr. | X | Y | Zn | $R^3$ | A B | E | physik. Daten |
|---|---|---|---|---|---|---|---|
| 53 | $CH_3$ | $CH_3$ | 6-$CH_3$ | $CH_3$ | $-(CH_2)_3-$ | H | |
| 54 | $CH_3$ | $CH_3$ | 6-$CH_3$ | $CH_3$ | $-(CH_2)_4-$ | H | Fp. 115-118°C |
| 55 | $CH_3$ | $CH_3$ | 6-$CH_3$ | $CH_2$ 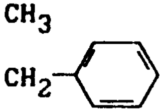 | $-(CH_2)_4-$ | H | Fp. 163-165°C |
| 56 | $CH_3$ | $CH_3$ | 6-$CH_3$ | $CH_2$ 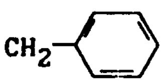 | $-(CH_2)_3-$ | H | Fp. 116-118°C |
| 57 | $CH_3$ | $CH_3$ | 6-$CH_3$ | 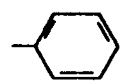 | $-(CH_2)_3-$ | H | |
| 58 | $CH_3$ | $CH_3$ | 6-$CH_3$ | 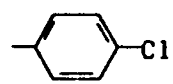 | $-(CH_2)_3-$ | H | Fp. 123-126°C |
| 59 | $CH_3$ | $CH_3$ | 6-$CH_3$ | $CHCl_2$ | $-(CH_2)_3-$ | H | |
| 60 | $CH_3$ | $CH_3$ | 6-$CH_3$ | 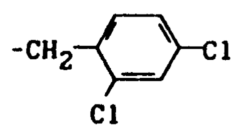 | $-(CH_2)_3-$ | H | |

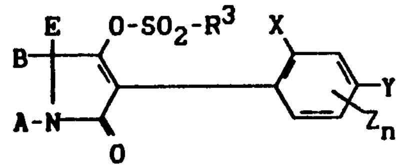

(Ib) (Fortsetzung)

| Beispiel-Nr. | X | Y | Zn | $R^3$ | A B | E | physik. Daten |
|---|---|---|---|---|---|---|---|
| 61 | $CH_3$ | $CH_3$ | 6-$CH_3$ | 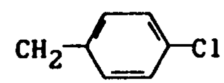 | $-(CH_2)_4-$ | H | Fp. 151-154°C |
| 62 | $CH_3$ | $CH_3$ | 6-$CH_3$ | 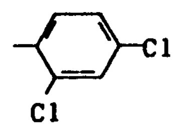 | $-(CH_2)_4-$ | H | |
| 63 | $CH_3$ | $CH_3$ | 6-$CH_3$ | n-$C_4H_9$ | $-(CH_2)_4-$ | H | |
| 64 | Cl | $CH_3$ | 6-Cl | $CH_3$ | $-(CH_2)_3-$ | H | |
| 65 | $CH_3$ | $CH_3$ | 5-$CH_3$ | $CH_3$ | $-(CH_2)_3-$ | H | |
| 66 | Cl | H | 6-Cl | n-$C_4H_9$ | $-(CH_2)_3-$ | H | |
| 67 | $CH_3$ | $CH_3$ | 5-$CH_3$ | n-$C_4H_9$ | $-(CH_2)_3-$ | H | |
| 68 | $CH_3$ | $CH_3$ | 6-$CH_3$ | n-$C_{12}H_{25}$ | $-(CH_2)_3-$ | H | Fp. 65°C |
| 69 | $CH_3$ | $CH_3$ | 6-$CH_3$ | n-$C_{12}H_{25}$ | $-(CH_2)_4-$ | H | Fp. 85-87°C |

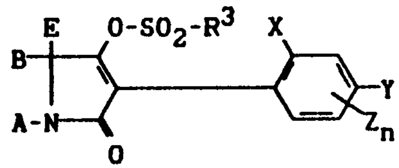

(Ib) (Fortsetzung)

| Beispiel-Nr. | X | Y | Zn | $R^3$ | A | B | E | physik. Daten |
|---|---|---|---|---|---|---|---|---|
| 70 | $CH_3$ | $CH_3$ | 6-$CH_3$ | 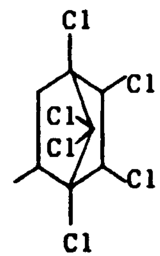 | -$(CH_2)_4$- | | H | |
| 71 | $CH_3$ | $CH_3$ | 6-$CH_3$ | 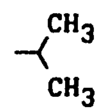 | -$(CH_2)_4$- | | H | |
| 72 | $CH_3$ | $CH_3$ | 6-$CH_3$ | $CH_3$ | 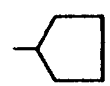 | H | H | |
| 73 | $CH_3$ | $CH_3$ | 6-$CH_3$ | $CH_3$ | 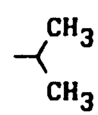 | H | H | |

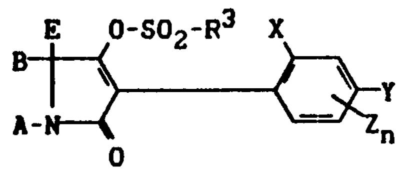 (Ib) (Fortsetzung)

| Beispiel-Nr. | X | Y | Zn | $R^3$ | A | B | E | physik. Daten |
|---|---|---|---|---|---|---|---|---|
| 74 | $CH_3$ | $CH_3$ | 6-$CH_3$ | n-$C_4H_9$ | 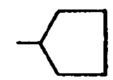 | H | H | |
| 75 | $CH_3$ | $CH_3$ | 6-$CH_3$ | n-$C_4H_9$ | 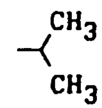 | H | H | |
| 76 | $CH_3$ | $CH_3$ | 6-$CH_3$ | 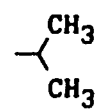 | -$(CH_2)_3$- | | H | |
| 77 | $CH_3$ | $CH_3$ | 6-$CH_3$ | 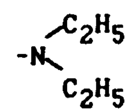 | -$(CH_2)_3$- | | H | |
| 78 | $CH_3$ | $CH_3$ | 6-$CH_3$ | 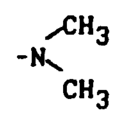 | -$(CH_2)_4$- | | H | |

Beispiel 79

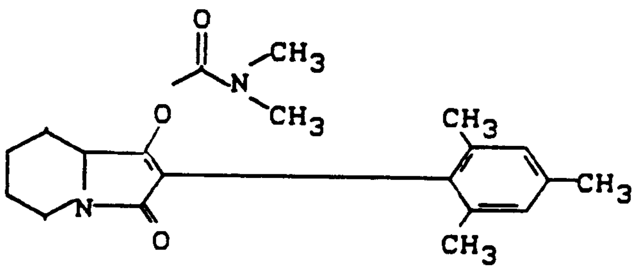

2,71 g (0,001 Mol) 3-(2,4,6-Trimethyl-phenyl)-1,5-tetramethylen-2,4-pyrrolidin-dion werden in 15 ml absolutem Dimethylformamid bei Raumtemperatur suspendiert. Man versetzt portionsweise mit 0,3 g (0,01 Mol) einer 80 %igen Natriumhydridsuspension in Paraffinöl. Es wird solange nachgerührt, bis die Wasserstoff-Entwicklung beendet ist. Dann wird auf 10 °C abgekühlt und mit 1,23 g (0,013 Mol) Dimethylcarbamidsäurechlorid versetzt. Man läßt auf Raumtemperatur kommen, erwärmt auf 80 °C und rührt 1 Stunde bei dieser Temperatur. Zur Aufarbeitung wird in 35 ml 1 %ige NaOH-Lösung eingerührt und 3 mal mit Methylchlorid extrahiert. Die organische Phase wird nacheinander mit 15 ml Wasser und 15 ml 5 %iger Salzsäure gewaschen und danach über Natriumsulfat getrocknet. Nach dem Abtrennen des Trockenmittels und abziehen des Lösungsmittels im Rotationsverdampfer wird das Produkt durch Chromatographie gereinigt. (Laufmittel: Essigester/Cyclohexan 1:5)
Ausbeute: 3,2 g (94 %) der Titelverbindung
Analog werden erhalten:

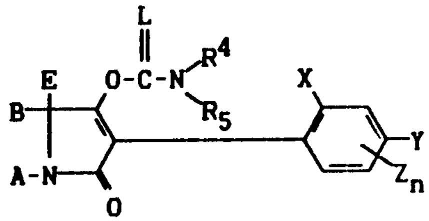

(Ic)

| Beispiel-Nr. | X | Y | Zn | L | $R^4$ | $R_5$ | A B | E | physik. Daten |
|---|---|---|---|---|---|---|---|---|---|
| 80 | $CH_3$ | $CH_3$ | 6-$CH_3$ | S | $CH_3$ | $CH_3$ | $-(CH_2)_4-$ | H | Fp. 188-189°C |
| 81 | Cl | H | 6-Cl | O | $CH_3$ | $CH_3$ | $-(CH_2)_4-$ | H | Fp. 119-121°C |
| 82 | Cl | H | 6-Cl | S | $CH_3$ | $CH_3$ | $-(CH_2)_4-$ | H | Fp. 158-161°C |
| 83 | $CH_3$ | $CH_3$ | 6-$CH_3$ | O | $CH_3$ | $CH_3$ | $-(CH_2)_3-$ | H | |
| 84 | $CH_3$ | $CH_3$ | 6-$CH_3$ | S | $CH_3$ | $CH_3$ | $-(CH_2)_3-$ | H | |
| 85 | $CH_3$ | $CH_3$ | 6-$CH_3$ | O | $-CH_2-CH=CH_2$ | $-CH_2-CH=CH_2$ | $-(CH_2)_3-$ | H | |
| 86 | $CH_3$ | $CH_3$ | 6-$CH_3$ | O | $CH_3$ | 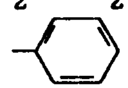 | $-(CH_2)_3-$ | H | |
| 87 | $CH_3$ | $CH_3$ | 6-$CH_3$ | O | $C_3H_7(n)$ | $C_3H_7(n)$ | $-(CH_2)_3-$ | H | |
| 88 | $CH_3$ | $CH_3$ | 6-$CH_3$ | O | $—(CH_2)_5—$ | | $-(CH_2)_3-$ | H | |
| 89 | $CH_3$ | $CH_3$ | 6-$CH_3$ | S | $-(CH_2)_2-O-(CH_2)_2-$ | | $-(CH_2)_3-$ | H | |

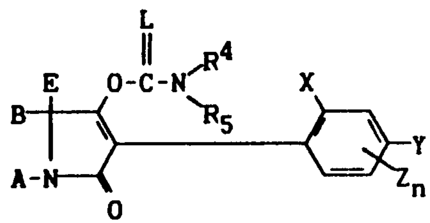

(Ic) (Fortsetzung)

| Beispiel-Nr. | X | Y | Zn | L | $R^4$ | $R_5$ | A B | E | physik. Daten |
|---|---|---|---|---|---|---|---|---|---|
| 90 | $CH_3$ | $CH_3$ | $6-CH_3$ | S | $-(CH_2)_5-$ | | $-(CH_2)_3-$ | H | Fp. 112-116°C |
| 91 | $CH_3$ | $CH_3$ | $6-CH_3$ | S | $C_2H_5$ | $C_2H_5$ | $-(CH_2)_3-$ | H | Fp. 112-116°C |
| 92 | $CH_3$ | $CH_3$ | $6-CH_3$ | S | $C_2H_5$ | 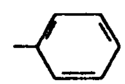 | $-(CH_2)_3-$ | H | Fp. 106-110°C |
| 93 | $CH_3$ | $CH_3$ | $6-CH_3$ | S | $-(-CH_2)_2-CH(CH_3)-(CH_2)_2-$ | | $-(CH_2)_3-$ | H | |

Beispiel 94

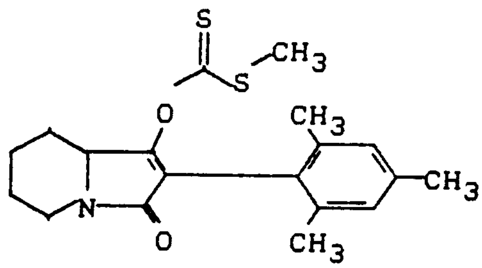

2,71 g (0,01 Mol) 3-(2,4,6-Trimethyl-phenyl)-1,5-tetramethylen-2,4-pyrrolidin-dion werden in 20 ml absolutem Dimethylformamid bei Raumtemperatur suspendiert. Man versetzt portionsweise mit 0,3 g einer 80 %igen Natriumhydridsuspension in Paraffinöl. Es wird solange nachgerührt, bis die Wasserstoff-Entwicklung beendet ist.

Dann werden 3 ml Schwefelkohlenstoff zugegeben und es wird drei Stunden bei Raumtemperatur gerrührt. Es werden drei ml Methyljodid zugegeben und anschließend wird weitere 3 Stunden bei Raumtemperatur gerührt.

Zur Aufarbeitung wird eine Mischung von 100 ml Wasser und 100 ml Methylenchlorid eingegossen, die wäßrige Phase wird abgetrennt und zweimal mit Methylenchlorid nachextrahiert. Die vereinigten organischen Phasen werden über Natriumsulfat getrocknet. Nach Abtrennen des Trockenmittels und Abziehen des Lösungsmittels wird chromatographiert.
(Laufmittel: Essigester/Cyclohexan 4:1)
Ausbeute: 1,5 g (41,5 %) der Titelverbindung

Beispiel 95

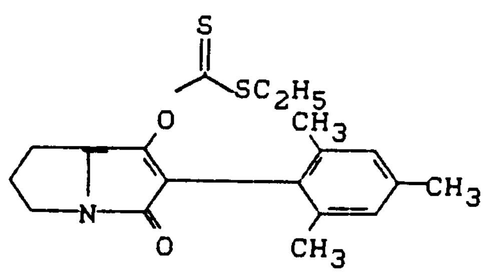

2,58 g (0,01 Mol) 3-(2,4,6-Trimethyl-phenyl)-1,5-trimethylen-2,4-pyrrolidin-dion werden in 20 ml absolutem Dimethylformamid bei Raumtemperatur suspendiert. Man versetzt portionsweise mit 0,3 g einer 80 %igen Natriumhydridsuspension in Paraffinöl. Es wird solange nachgerührt, bis die Wasserstoff-Entwicklung beendet ist.

Dann werden langsam 1,5 ml (0,014 Mol) Chlorthioameisensäure-S-ethylester zugetropft. Nach beendeter Zugabe wird noch 1 Stunde bei Raumtemperatur gerührt, dann wird mit 100 ml Wasser versetzt, 3 mal mit Methylenchlorid extrahiert, die organische Phase 1 mal mit 40 ml 5 %iger Salzsäure gewaschen, getrocknet und eingeengt.
Ausbeute: 1,34 g (38,8 %)
Analog werden erhalten:

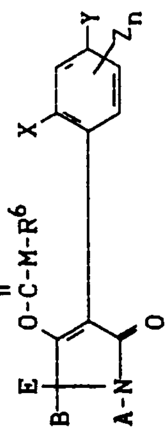

(Id)

| Beispiel-Nr. | X | Y | Zn | L | M | $R_6$ | A B | E | physik. Daten |
|---|---|---|---|---|---|---|---|---|---|
| 96 | $CH_3$ | $CH_3$ | 6-$CH_3$ | S | O | $C_2H_5$ | $-(CH_2)_3-$ | H | |
| 97 | $CH_3$ | $CH_3$ | 6-$CH_3$ | O | S | $C_2H_5$ | $-(CH_2)_3-$ | H | |
| 98 | $CH_3$ | $CH_3$ | 6-$CH_3$ | O | S | $C_2H_5$ | $-(CH_2)_4-$ | H | |
| 99 | $CH_3$ | $CH_3$ | 6-$CH_3$ | S | S | $C_2H_5$ | $-(CH_2)_4-$ | H | |
| 100 | $CH_3$ | $CH_3$ | 6-$CH_3$ | S | S | i-$C_3H_7$ | $-(CH_2)_4-$ | H | |
| 101 | $CH_3$ | $CH_3$ | 6-$CH_3$ | S | S | n-$C_4H_9$ | $-(CH_2)_4-$ | H | |
| 102 | $CH_3$ | $CH_3$ | 6-$CH_3$ | S | S | $-CH_2-$ 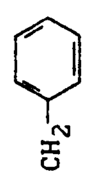 | $-(CH_2)_4-$ | H | Fp. 140° C |
| 103 | $CH_3$ | $CH_3$ | 6-$CH_3$ | S | S | $-CH_2-CH=CH_2$ | $-(CH_2)_4-$ | H | |
| 104 | $CH_3$ | $CH_3$ | 6-$CH_3$ | S | S | $-CH_2-C≡CH$ | $-(CH_2)_4-$ | H | Fp. 92-94° C |
| 105 | $CH_3$ | $CH_3$ | 6-$CH_3$ | S | S | $-CH_2-CH=CH_2$ | $-(CH_2)_3-$ | H | Fp. 103-105° C |
| 106 | $CH_3$ | $CH_3$ | 6-$CH_3$ | S | S | $C_2H_5$ | $-(CH_2)_3-$ | H | |
| 107 | $CH_3$ | $CH_3$ | 6-$CH_3$ | S | S | $CH_3$ | 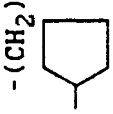 H | H | Fp. 141-142° C |

Die Wirkstoffe eignen sich bei guter Pflanzenverträglichkeit und günstiger Warmblütertoxizität zur Bekämpfung von tierischen Schädlingen, insbesondere der Klasse Arachuida und der Ordnung Milben (Acarina), die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma

spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben.

Sie sind gegen normalsensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe akarizide Wirksamkeit aus. Sie lassen sich mit besonders gutem Erfolg gegen pflanzenschädigende Milben, wie beispielsweise gegen die gemeine Spinnmilbe (Tetranychus urticae) einsetzen.

Die erfindungsgemäßen Wirkstoffe können weiterhin als Defoliants, Desiccants, Krautabtötungsmittel und insbesondere als Unkrautvernichtungsmittel verwendet werdend. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Charakteristisch für die erfindungsgemäßen Verbindungen ist, daß sie eine selektive Wirksamkeit gegen monokotyle Unkräuter im Vor- und Nachlaufverfahren (Pre- und Postemergence) bei guter Kulturflanzenverträglichkeit aufweisen.

Die erfindungsgemäßen Wirkstoffe können z.B. bei den folgenden Pflanzen verwendet werden:

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer hervorragenden Wirkung gegen Schadpflanzen gute Verträglichkeit gegenüber wichtigen Kulturpflanzen, wie z.B. Weizen, Baumwolle, Sojabohnen, Citrusfrüchten und Zuckerrüben, und können daher als selektive Unkrautbekämpfungsmittel eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u-ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B.

gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Herbiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäß verwendbaren Wirkstoffe eignen sich auch zur Bekämpfung von Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

## Beispiel A

Phaedon-Larven-Test

| | |
|---|---|
| Lösungsmittel: | 7 Gewichtsteile Dimethylformamid/P-Verb.: Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: (1), (2), (4), (59).

Beispiel B

Plutella-Test

Lösungsmittel: 7 Gewichtsteile Dimethylformamid/P-Verb.: Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: (2)

Beispiel C

Nephotettix-Test

Lösungsmittel: 7 Gewichtsteile Dimethylformamid/P-Verb.: Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: (1, (4), (53), (59).

Beispiel D

Spodoptera-Test

Lösungsmittel: 7 Gewichtsteile Dimethylformamid/P-Verb.: Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: (2).

Beispiel E

Pre-emergence-Test

Lösungsmittel: 7 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle

100 % = totale Vernichtung

Bei diesem Test zeigen z.B. die folgenden Verbindungen der Herstellungsbeispiele überlegene Wirksamkeit gegenüber dem Stand der Technik: (1), (4), (53), (56), (58), (60).

Beispiel F

Post-emergence-Test

Lösungsmittel: 7 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden, Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle

100 % = totale Vernichtung

Bei diesem Test zeigen z.B. die folgenden Verbindungen der Herstellungsbeispiele überlegene Wirksamkeit gegenüber dem Stand der Technik: (1), (4), (53), (60), (85), (88).

**Patentansprüche**

1. 3-Arylpyrrolidin-2,4-dion-Derivate der Formel (I)

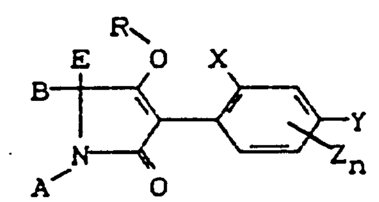

(I)

in welcher

A für gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann oder gegebenenfalls durch Halogen, $C_1$-$C_6$-Alkyl, $C_1$-$C_6$-Haloalkyl, $C_1$-$C_6$-Alkoxy, Nitro substituiertes Aryl-$C_1$-$C_6$-alkyl steht,

B und E unabhängig voneinander für Wasserstoff, geradkettiges oder verzweigtes $C_1$-$C_{12}$-Alkyl, $C_1$-$C_8$-Alkoxyalkyl stehen

oder

A und B mit dem Stickstoff- bzw. Kohlenstoffatom, an das sie gebunden sind, einen 4 bis 8-gliedrigen Zyklus bilden, der durch ein Schwefelatom, eine Sulfoxid- oder eine Sulfonylgruppe unterbrochen sein kann,

X für $C_1$-$C_6$-Alkyl, Halogen oder $C_1$-$C_6$-Alkoxy steht,

Y für Wasserstoff, $C_1$-$C_6$-Alkyl, Halogen, $C_1$-$C_6$-Alkoxy, $C_1$-$C_3$-Halogenalkyl steht,

Z für $C_1$-$C_6$-Alkyl, Halogen, $C_1$-$C_6$-Alkoxy steht,
n für eine Zahl von 0 - 3 steht,
R für die Gruppen

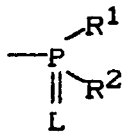 (a) $-SO_2-R^3$ (b)

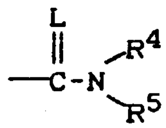 (c) oder 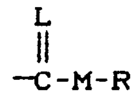 (d)

steht,
in welchen
L und M jeweils für Sauerstoff oder Schwefel stehen,
$R^1$, $R^2$ und $R^3$ unabhängig voneinander für gegebenenfalls durch Halogen substituiertes $C_1$-$C_8$-Alkyl, $C_1$-$C_8$-Alkoxy, $C_1$-$C_8$-Alkylamino, Di-$C_1$-$C_8$)-Alkylamino, $C_1$-$C_8$-Alkylthio, $C_2$-$C_5$-Alkenylthio, $C_2$-$C_5$-Alkinylthio, $C_3$-$C_7$-Cycloalkylthio, für gegebenenfalls durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl, $C_1$-$C_4$-Halogenalkyl substituiertes Phenyl, Benzyl, Phenoxy oder Phenylthio stehen,
$R^4$ und $R^5$ unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_{20}$-Halogenalkyl, $C_1$-$C_{20}$-Alkyl oder $C_1$-$C_{20}$-Alkoxy substituiertes Phenyl, für gegebenenfalls durch Halogen, $C_1$-$C_{20}$-Alkyl, $C_1$-$C_{20}$-Halogenalkyl oder $C_1$-$C_{20}$-Alkoxy substituiertes Benzyl steht oder zusammen für einen gegebenenfalls durch Sauerstoff unterbrochenen $C_2$-$C_6$-Alkylenring stehen,
$R^6$ für gegebenenfalls durch Halogen substituiertes $C_1$-$C_{20}$-Alkyl, das durch Sauerstoff unterbrochen sein kann für gegebenenfalls durch Halogen, $C_1$-$C_{20}$-Haloganalkyl, $C_1$-$C_{20}$-Alkoxy substituiertes Phenyl, für gegebenenfalls durch Halogen, $C_1$-$C_{20}$-Halogenalkyl oder $C_1$-$C_{20}$-Alkoxy substituiertes Benzyl, für $C_2$-$C_8$-Alkenyl oder für $C_2$-$C_5$-Alkinyl steht,

sowie die enantiomerenreinen Formen in Verbindungen der Formel (I), mit der Maßgabe, daß L und M nicht gleichzeitig für Sauerstoff stehen.

**2.** 3-Arylpyrrolidin-2,4-dion-Derivate der Formel (I) gemäß Anspruch 1,
in welcher
A für gegebenenfalls durch Halogen substituiertes geradkettiges oder verzweigtes $C_1$-$C_{10}$-Alkyl, $C_3$-$C_6$-Alkenyl, $C_3$-$C_6$-Alkinyl, $C_1$-$C_8$-Alkoxy-$C_2$-$C_8$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3-7 Ringatomen, das durch 1-2 Sauerstoff und/oder Schwefelatomen unterbrochen sein kann oder gegebenenfalls durch Halogen , $C_1$-$C_4$-Alkyl , $C_1$-$C_4$-Haloalkyl, $C_1$-$C_4$-Alkoxy , Nitro substituiertes Aryl-$C_1$-$C_4$-alkyl steht,
B, E unabhängig voneinander für Wasserstoff, geradkettiges oder verzweigtes $C_1$-$C_{10}$-Alkyl, $C_1$-$C_6$-Alkoxyalkyl stehen
oder
A und B mit dem Stickstoff- bzw. Kohlenstoffatom, an das sie gebunden sind, einen 4 bis 7-gliedrigen Zyklus bilden, der durch ein Schwefelatom, oder durch eine Sulfoxid- bzw. Sulfonylgruppe unterbrochen sein kann,
X für $C_1$-$C_4$-Alkyl, Halogen oder $C_1$-$C_4$-Alkoxy steht,
Y für Wasserstoff, $C_1$-$C_6$-Alkyl, Halogen, $C_1$-$C_4$-Alkoxy, $C_1$-$C_2$-Halogenalkyl steht,
Z für $C_1$-$C_4$-Alkyl, Halogen, $C_1$-$C_4$-Alkoxy steht,
n für eine Zahl von 0 - 3 steht,
R für die Gruppen

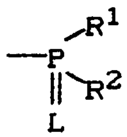 (a) $-SO_2-R^3$ (b)

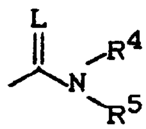 (c) 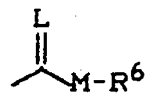 (d)

steht

in welchen

- L und M — jeweils für Sauerstoff oder Schwefel stehen,
- $R^1$, $R^2$ und $R^3$ — unabhängig voneinander für gegebenenfalls durch Halogen substituiertes $C_1$-C6-Alkyl, $C_1$-$C_6$-Alkoxy, $C_1$-$C_6$-Alkylamino, Di-($C_1$-$C_6$)-Alkylamino, $C_1$-$C_6$-Alkylthio, $C_3$-$C_4$-Alkenylthio, $C_2$-$C_4$-Alkinylthio, $C_3$-$C_6$-Cycloalkylthio, für gegebenenfalls durch Fluor, Chlor, Brom, Nitro, Cyano, $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkyl, $C_1$-$C_3$-Halogenalkyl substituiertes Phenyl, Benzyl, Phenoxy oder Phenylthio stehen,
- $R^4$ und $R^5$ — unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_5$-Halogenalkyl, $C_1$-$C_5$-Alkyl oder $C_1$-$C_5$-Alkoxy substituiertes Phenyl, für gegebenenfalls durch Halogen, $C_1$-$C_5$-Alkyl, $C_1$-$C_5$-Halogenalkyl oder $C_1$-$C_5$-Alkoxy substituiertes Benzyl steht,
- $R^6$ — für gegebenenfalls durch Halogen substituiertes $C_1$-$C_{20}$-Alkyl, das durch Sauerstoff unterbrochen sein kann für gegebenenfalls durch Halogen, $C_1$-$C_5$-Halogenalkyl oder $C_1$-$C_5$-Alkoxy substituiertes Phenyl, oder für gegebenenfalls durch Halogen, $C_1$-$C_5$-Halogenalkyl oder $C_1$-$C_5$-Alkoxy substituiertes Benzyl steht,

sowie die enantiomerenreinen Formen von Verbindungen der Formel (I), mit der Maßgabe, daß L und M nicht gleichzeitig für Sauerstoff stehen.

**3.** 3-Arylpyrrolidin-2,4-dion-Derivate der Formel (I) gemäß Ansprch 1,

in welcher

- A — für gegebenenfalls durch Halogen substituiertes geradkettiges oder verzweigtes $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3-6 Ringatomen, das durch 1-2 Sauerstoff- und/oder Schwefelatomen unterbrochen sein kann oder gegebenenfalls durch Fluor-, Chlor-, Brom-, Methyl-, Ethyl-, Propyl-, iso-Propyl-, Methoxy-, Ethoxy-, Trifluormethyl-, Nitro substituiertes Aryl-$C_1$-$C_3$-alkyl steht,
- B, E — unabhängig voneinander für Wasserstoff,-geradkettiges oder verzweigtes $C_1$-$C_8$-Alkyl, $C_1$-$C_4$-Alkoxyalkyl stehen,

oder

- A und B — mit dem Stickstoff- bzw. Kohlenstoffatom an das sie gebunden sind, einen 5-6-gliedrigen Zyklus bilden, der durch ein Schwefelatom oder durch eine Sulfoxid- bzw, Sulfonylgruppe unterbrochen sein kann,
- X — für Methyl, Ethyl, Propyl, i-Propyl, Fluor, Chlor, Brom, Methoxy oder Ethoxy steht,
- Y — für Wasserstoff, Methyl, Ethyl, Propyl, i-Propyl, Butyl, i-Butyl, tert.-Butyl, Fluor, Chlor, Brom, Methoxy, Ethoxy oder Trifluormethyl steht,
- Z — für Methyl, Ethyl, i-Propyl, Butyl, i-Butyl, tert.-Butyl, Fluor, Chlor, Brom, Methoxy oder Ethoxy steht,
- n — für eine Zahl von 0-3 steht,
- R — für die Gruppen

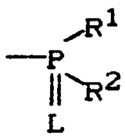 (a) $-SO_2-R^3$ (b)

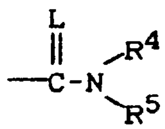 (c) oder 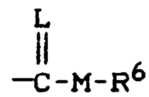 (d)

steht,
in welchen

L und M jeweils für Sauerstoff oder Schwefel stehen,

$R^1$, $R^2$ und $R^3$ unabhängig voneinander für gegebenenfalls durch Fluor oder Chlor substituiertes $C_1$-$C_4$-Alkyl, $C_1$-$C_4$-Alkoxy, $C_1$-$C_4$-Alkylamino, Di-($C_1$-$C_4$-Alkyl)-amino, $C_1$-$C_4$-Alkylthio, für gegebenenfalls durch Fluor, Chlor, Brom, Nitro, Cyano, $C_1$-$C_2$-Alkoxy, $C_1$-$C_4$-Fluoralkoxy, $C_1$-$C_2$-Chloralkoxy, $C_1$-$C_2$-Alkylthio, $C_1$-$C_2$-Fluoralkylthio, $C_1$-$C_2$-Chloralkylthio, $C_1$-$C_3$-Alkyl substituiertes Phenyl, Benzyl, Phenoxy oder Phenylthio stehen,

$R^4$ und $R^5$ unabhängig voneinander für gegebenenfalls durch Fluor, Chlor, Brom substituiertes $C_1$-$C_{10}$-Alkyl, $C_1$-$C_{10}$-Alkoxy, $C_1$-$C_{10}$-Alkoxy-($C_1$-$C_{10}$)-alkyl, für gegebenenfalls durch Fluor, Chlor, Brom, $C_1$-$C_{20}$-Halogenalkyl, $C_1$-$C_{20}$-Alkyl oder $C_1$-$C_4$-Alkoxy substituiertes Phenyl, für gegebenenfalls durch Fluor, Chlor, Brom, $C_1$-$C_4$-Alkyl, $C_1$-$C_4$-Halogenalkyl oder $C_1$-$C_4$-Alkoxy substituiertes Benzyl stehen,

$R^6$ für gegebenenfalls durch Fluor, Chlor, Brom, substituiertes $C_1$-$C_{10}$-Alkyl, das durch Sauerstoff unterbrochen sein kann, für gegebenenfalls durch Fluor, Chlor, Brom, $C_1$-$C_4$-Halogenalkyl, $C_1$-$C_4$-Alkoxy substituiertes Phenyl oder für gegebenenfalls durch Fluor, Chlor, Brom, $C_1$-$C_4$-Halogenalkyl oder $C_1$-$C_4$-Alkoxy substituiertes Benzyl steht,

sowie die enantiomerenreinen Formen von Verbindungen der Formel (I), mit der Maßgabe, daß L und M nicht gleichzeitig für Sauerstoff stehen.

**4.** Verfahren zur Herstellung von 3-Arylpyrrolidin-2,4-dion-Derivaten der Formel (I) gemäß Anspruch 1, dadurch gekennzeichnet, daß man zum Erhalt von Verbindungen der Struktur (Ia)

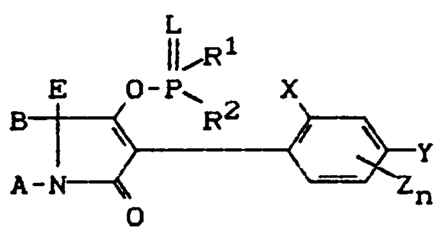 (Ia)

in welcher

A, B, E, L, X, Y, Z, $R^1$, $R^2$ und n die in Anspruch 1 angegebene Bedeutung haben,

3-Aryl-pyrrolidin-2,4-dione der Formel (II) bzw. deren Enole

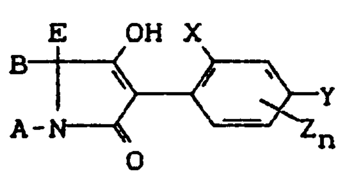 (II)

in welcher
A, B E, X, Y, Z und n die in Anspruch 1 angegebene Bedeutung haben,
mit Phosphorverbindungen der allgemeinen Formel (III)

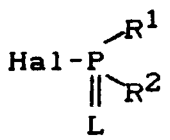

(III)

in welcher
L, $R^1$ und $R^2$ die in Anspruch 1 angegebene Bedeutung haben
und
Hal für Halogen steht,
gegebenenfalls in Gegenwart eines Verdünnungsmittels und gegebenenfalls in Gegenwart eines Säurebindemittels umsetzt,
oder daß man zum Erhalt von Verbindungen der Formel (Ib)

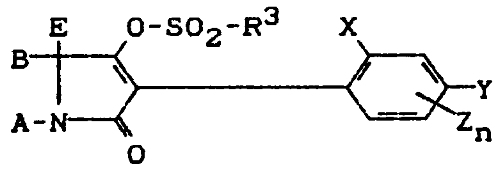

(Ib)

in welcher
A, B, E, X, Y, Z, $R^3$ und n die in Anspruch 1 angegebene Bedeutung haben,
Verbindungen der Formel (II)

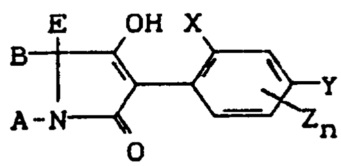

(II)

in welcher
A, B, E, X, Y, Z und n die in Anspruch 1 angegebene Bedeutung haben
mit Sulfonsäurechloriden der allgemeinen Formel (IV)

$R^3$-$SO_2$-Cl (IV)

in welcher
$R^3$ die in Anspruch 1 angegebene Bedeutung hat
gegebenenfalls in Gegenwart eines Verdünnungsmittels und gegebenenfalls in Gegenwart eines Säurebindemittels,
umsetzt,
oder, daß man zum Erhalt von Verbindungen der Formel (Ic)

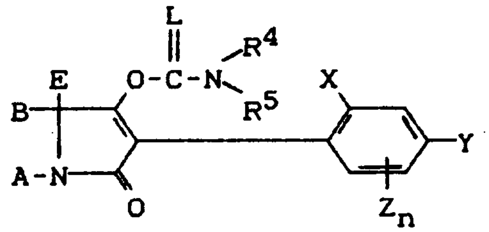

(Ic)

in welcher

A, B, E, L, X, Y, Z, $R^4$, $R^5$ und n die in Anspruch 1 angegebene Bedeutung haben,

Verbindungen der Formel (II),

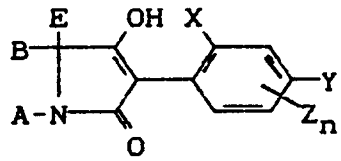

(II)

in welcher

A, B, E, X, Y, Z und n die in Anspruch 1 angegebene Bedeutung haben

α) mit Isocyanaten der allgemeinen Formel (V)

$R^4$-N = C = O (V)

in welcher

$R^4$ die in Anspruch 1 angegebene Bedeutung hat

gegebenenfalls in Gegenwart eines Verdünnungsmittels und gegebenenfalls in Gegenwart eines Katalysators umsetzt

oder

β) mit Carbamidsäurechloriden oder Thiocarbamidsäurechloriden der allgemeinen Formel (VI)

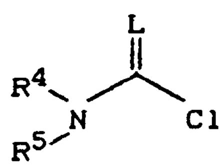

(VI)

in welcher

L, $R^4$ und $R^5$ die in Anspruch 1 angegebene Bedeutung haben

gegebenenfalls in Gegenwart eines Verdünnungsmittels und gegebenenfalls in Gegenwart eines Säurebindemittels,

umsetzt,

oder daß man zum Erhalt von Verbindungen der Formel (Id)

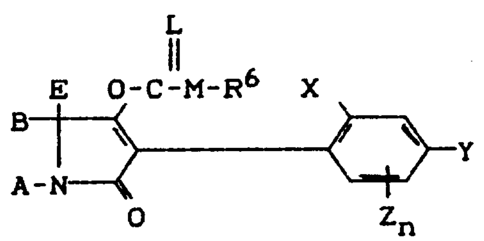

(Id)

in welcher

A, B, E, L, M, $R^6$, X, Y, Z und n die in Anspruch 1 angegebene Bedeutung haben,
Verbindungen der Formel (II)

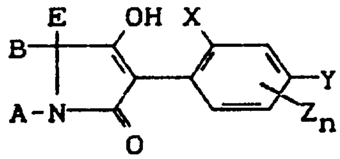

(II)

in welcher

A, B, E, X, Y, Z und n die in Anspruch 1 angegebene Bedeutung haben

α) mit Chlormonothioameisensäureestern oder Chlordithioameisensäureestern der allgemeinen Formel VII

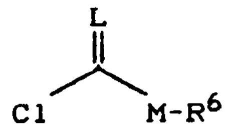

(VII)

in welcher

L, M, $R^6$ die in Anspruch 1 angegebene Bedeutung haben

gegebenenfalls in Gegenwart eines Verdünnungsmittels und gegebenenfalls in Gegenwart eines Säurebindemittels umsetzt,

oder

β) mit Schwefelkohlenstoff und anschließend mit Alkylhalogeniden der allgemeinen Formel VIII

$R^6$-Hal (VIII)

in welcher

$R^6$ die in Anspruch 1 angegebene Bedeutung hat

und

Hal für Chlor, Brom, Jod

steht,

umsetzt.

oder, daß man zum Erhalt von Verbindungen der Formel (Ie)

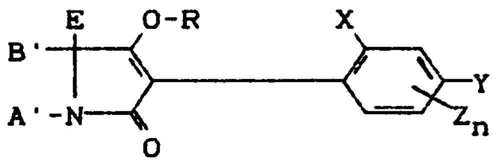

(Ie)

in welcher

R, X, Y, Z und n die in Anspruch 1 angegebene Bedeutung haben,

E für Wasserstoff steht

und

A', B' zusammen für $-CH_2-CH_2-SO-CH_2-$,$-CH_2-SO-CH_2-CH_2-$ oder $-CH_2-SO-CH_2-$stehen,

Verbindungen der Formel (If)

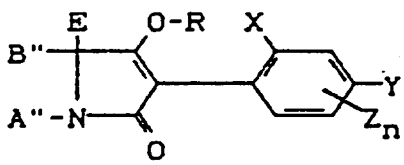

(If)

in welcher

R, X, Y, Z und n die in Anspruch 1 angegebene Bedeutung haben

E für Wasserstoff steht

und

A'', B'' zusammen für $-CH_2-CH_2-S-CH_2-$,$-CH_2-S-CH_2-CH_2-$ oder $-CH_2-S-CH_2-$ stehen,

mit annähernd äquimolaren Mengen eines Oxidationsmittels gegebenenfalls in Gegenwart eines Verdünnungsmittels umsetzt,

oder, daß man zum Erhalt von Verbindungen der Formel (Ig)

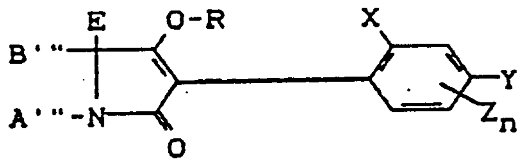

(Ig)

in welcher

R, X, Y, Z und n die in Anspruch 1 angegebene Bedeutung haben,

E für Wasserstoff steht und

A''', B''' zusammen für $-CH_2-CH_2-SO_2-CH_2-$, $-CH_2-SO_2-CH_2-CH_2-$ oder $-CH_2-SO_2-CH_2-$stehen,

Verbindungen der Formel (If)

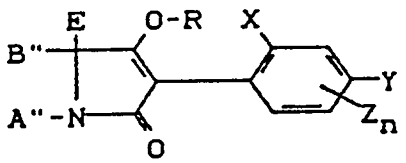

(If)

in welcher

R, X, Y, Z und n die in Anspruch 1 angegebenen Bedeutung haben

E für Wasserstoff steht

und

A'', B'' zusammen für $-CH_2-CH_2-S-CH_2-$, $-CH_2-S-CH_2-CH_2-$ und $-CH_2-S-CH_2-$ stehen,

mit mindestens doppelt äquimolaren Mengen eines Oxidationsmittels, gegebenenfalls in Gegenwart eines Verdünnungsmittels umsetzt.

**5.** Insektizide, akarizide und herbizide Mittel, gekennzeichnet durch einen Gehalt an mindestens einem 3-Arylpyrrolidin-2,4-dion-Derivat der Formel (I).

**6.** Verfahren zur Bekämpfung von Insekten und/oder Spinnentieren und/oder Unkräutern, dadurch gekennzeichnet, daß man 3-Arylpyrrolidin-2,4-dion-Derivate der Formel (I) auf Insekten und/oder Spinnentiere und/oder Unkräuter und/oder deren Lebensraum einwirken läßt.

**7.** Verwendung von 3-Arylpyrrolidin-2,4-dion-Derivaten der Formel (I) zur Bekämpfung von Insekten und/oder Spinnentieren und/oder Unkräutern.

**8.** Verfahren zur Herstellung von insektiziden und/oder akariziden und/oder herbiziden Mitteln, dadurch gekennzeichnet, daß man 3-Arylpyrrolidin-2,4-dion-Derivate der Formel (I), mit Streckmitteln und/oder oberflächenaktiven Mitteln vermischt.

## Claims

**1.** 3-Arylpyrrolidine-2,4-dione derivatives of the formula (I)

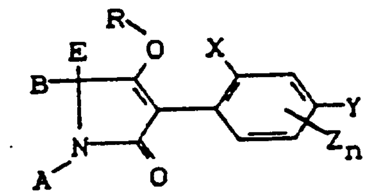

(I)

in which

| | |
|---|---|
| A | represents optionally halogen-substituted straight-chain or branched $C_1$-$C_{12}$-alkyl, $C_3$-$C_8$-alkenyl, $C_3$-$C_8$-alkinyl, $C_1$-$C_{10}$-alkoxy-$C_2$-$C_8$-alkyl, $C_1$-$C_8$-polyalkoxy-$C_2$-$C_8$-alkyl, $C_1$-$C_{10}$-alkylthio-$C_2$-$C_8$-alkyl, cycloalkyl which has 3-8 ring atoms and which can be interrupted by oxygen and/or sulphur, or represents aryl-$C_1$-$C_6$-alkylwhich is optionally substituted by halogen, $C_1$-$C_6$-alkyl, $C_1$-$C_6$-haloalkyl, $C_1$-$C_6$-alkoxy or nitro, |
| B and E | independently of one another represent hydrogen or straight-chain or branched $C_1$-$C_{12}$-alkyl or $C_1$-$C_8$-alkoxyalkyl |

or

| | |
|---|---|
| A and B | together with the nitrogen or carbon atom to which they are bonded form a 4 to 8-membered cycle which can be interrupted by a sulphur atom or a sulphoxide or a sulphonyl group, |
| X | represents $C_1$-$C_6$-alkyl, halogen or $C_1$-$C_6$-alkoxy, |
| Y | represents hydrogen, $C_1$-$C_6$-alkyl, halogen, $C_1$-$C_6$-alkoxy or $C_1$-$C_3$-halogenoalkyl, |
| Z | represents $C_1$-$C_6$-alkyl, halogen or $C_1$-$C_6$-alkoxy, |
| n | represents a number from 0 - 3, |
| R | represents the groups |

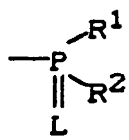 (a) $-SO_2-R^3$ (b)

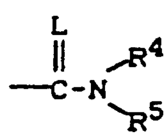 (c) or 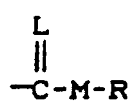 (d)

in which

| | |
|---|---|
| L and M | in each case represent oxygen or sulphur, |
| $R^1$, $R^2$ and $R^3$ | independently of one another represent $C_1$-$C_8$-alkyl, $C_1$-$C_8$-alkoxy, $C_1$-$C_8$-alkylamino, di-($C_1$-$C_8$)-alkylamino, $C_1$-$C_8$-alkylthio, $C_2$-$C_5$-alkenylthio, $C_2$-$C_5$-alkinylthio or $C_3$-$C_7$-cycloalkylthio, each of which is optionally substituted by halogen, or represent phenyl, benzyl, phenoxy or phenylthio, each of which is optionally substituted by halogen, nitro, cyano, $C_1$-$C_4$-alkoxy, $C_1$-$C_4$-halogenoalkoxy, $C_1$-$C_4$-alkylthio, $C_1$-$C_4$-halogenoalkylthio, $C_1$-$C_4$-alkyl or $C_1$-$C_4$-halogenoalkyl, |
| $R^4$ and $R^5$ | independently of one another represents $C_1$-$C_{20}$-alkyl, $C_1$-$C_{20}$-alkoxy, $C_2$-$C_8$-alkenyl or $C_1$-$C_{20}$-alkoxy-$C_1$-$C_{20}$-alkyl, each of which is optionally substituted by halogen, or represents phenyl which is optionally substituted by halogen, $C_1$-$C_{20}$-halogenoalkyl, $C_1$-$C_{20}$-alkyl or $C_1$-$C_{20}$-alkoxy, or represents benzyl which is optionally substituted by halogen, $C_1$-$C_{20}$-alkyl, $C_1$-$C_{20}$-halogenoalkyl or $C_1$-$C_{20}$-alkoxy, or together represent a $C_2$-$C_6$-alkylene ring which is optionally interrupted by oxygen, |

R⁶ represents $C_1$-$C_{20}$-alkyl which is optionally substituted by halogen and which can be interrupted by oxygen, or represents phenyl which is optionally substituted by halogen, $C_1$-$C_{20}$-halogenoalkyl or $C_1$-$C_{20}$-alkoxy, or represents benzyl which is optionally substituted by halogen, $C_1$-$C_{20}$-halogenoalkyl or $C_1$-$C_{20}$-alkoxy, or represents $C_2$-$C_8$-alkenyl, or represents $C_2$-$C_5$-alkinyl,

as well as the pure enantiomeric forms in compounds of the formula (I), with the proviso that L and M do not simultaneously represent oxygen.

**2.** 3-Arylpyrrolidine-2,4-dione derivatives of the formula (I) according to Claim 1, in which

A represents optionally halogen-substituted straight-chain or branched $C_1$-$C_{10}$-alkyl, $C_3$-$C_6$-alkenyl, $C_3$-$C_6$-alkinyl, $C_1$-$C_8$-alkoxy-$C_2$-$C_8$-alkyl, $C_1$-$C_6$-polyalkoxy-$C_2$-$C_6$-alkyl, $C_1$-$C_8$-alkylthio-$C_2$-$C_6$-alkyl, cycloalkyl which has 3-7 ring atoms and which can be interrupted by 1-2 oxygen and/or sulphur atoms, or represents aryl-$C_1$-$C_4$-alkyl which is optionally substituted by halogen , $C_1$-$C_4$-alkyl , $C_1$-$C_4$-haloalkyl, $C_1$-$C_4$-alkoxy or nitro,

B and E independently of one another represent hydrogen or straight-chain or branched $C_1$-$C_{10}$-alkyl or $C_1$-$C_6$-alkoxyalkyl,

or

A and B together with the nitrogen or carbon atom to which they are bonded form a 4 to 7-membered cycle which can be interrupted by a sulphur atom, or by a sulphoxide or sulphonyl group,

X represents $C_1$-$C_4$-alkyl, halogen or $C_1$-$C_4$-alkoxy,

Y represents hydrogen, $C_1$-$C_6$-alkyl, halogen, $C_1$-$C_4$-alkoxy or $C_1$-$C_2$-halogenoalkyl,

Z represents $C_1$-$C_4$-alkyl, halogen or $C_1$-$C_4$-alkoxy,

n represents a number from 0-3,

R represents the groups

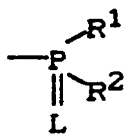 (a)    $-SO_2-R^3$ (b)

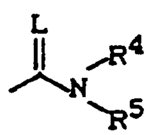 (c)    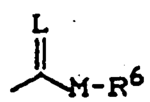 (d)

in which

L and M in each case represent oxygen or sulphur,

$R^1$, $R^2$ and $R^3$ independently of one another represent $C_1$-$C_6$-alkyl, $C_1$-$C_6$-alkoxy, $C_1$-$C_6$-alkylamino, di-($C_1$-$C_6$)-alkylamino, $C_1$-$C_6$-alkylthio, $C_3$-$C_4$-alkenylthio, $C_2$-$C_4$-alkinylthio or $C_3$-$C_6$-cycloalkylthio, each of which is optionally substituted by halogen, or represent phenyl, benzyl, phenoxy or phenylthio, each of which is optionally substituted by fluorine, chlorine, bromine, nitro, cyano, $C_1$-$C_3$-alkoxy, $C_1$-$C_3$-halogenoalkoxy, $C_1$-$C_3$-alkylthio, $C_1$-$C_3$-halogenoalkylthio, $C_1$-$C_3$-alkyl or $C_1$-$C_3$-halogenoalkyl,

$R^4$ and $R^5$ independently of one another represent $C_1$-$C_{20}$-alkyl, $C_1$-$C_{20}$-alkoxy, $C_2$-$C_2$-alkenyl or $C_1$-$C_{20}$-alkoxy-$C_1$-$C_{20}$-alkyl, each of which is optionally substituted by halogen, or represent phenyl which is optionally substituted by halogen, $C_1$-$C_5$-halogenoalkyl, $C_1$-$C_5$-alkyl or $C_1$-$C_5$-alkoxy, or represent benzyl which is optionally substituted by halogen, $C_1$-$C_5$-alkyl, $C_1$-$C_5$-halogenoalkyl or $C_1$-$C_5$-alkoxy,

$R^6$ represents $C_1$-$C_{20}$-alkyl which is optionally substituted by halogen and which can be interrupted by oxygen, or represents phenyl which is optionally substituted by halogen, $C_1$-$C_5$-halogenoalkyl or $C_1$-$C_5$-alkoxy, or represents benzyl which is optionally substituted by halogen, $C_1$-$C_5$-halogenoalkyl or $C_1$-$C_5$-alkoxy,

as well as the pure enantiomeric forms of compounds of the formula (I), with the proviso that L and M

do not simultaneously represent oxygen.

3. 3-Arylpyrrolidine-2,4-dione derivatives of the formula (I) according to Claim 1,
in which

A represents optionally halogen-substituted straight-chain or branched $C_1$-$C_8$-alkyl, $C_3$-$C_4$-alkenyl, $C_3$-$C_4$-alkinyl, $C_1$-$C_6$-alkoxy-$C_2$-$C_4$-alkyl, $C_1$-$C_4$-polyalkoxy-$C_2$-$C_4$-alkyl, $C_1$-$C_6$-alkylthio-$C_2$-$C_4$-alkyl, cycloalkyl which has 3-6 ring atoms and which can be interrupted by 1-2 oxygen and/or sulphur atoms, or represents aryl-$C_1$-$C_3$-alkyl which is optionally substituted by fluorine, chlorine, bromine, methyl, ethyl, propyl, iso-propyl, methoxy, ethoxy, trifluoromethyl or nitro,

B and E independently of one another represent hydrogen or straight-chain or branched $C_1$-$C_8$-alkyl or $C_1$-$C_4$-alkoxyalkyl,

or

A and B together with the nitrogen or carbon atom to which they are bonded form a 5-6-membered cycle which can be interrupted by a sulphur atom or by a sulphoxide or sulphonyl group,

X represents methyl, ethyl, propyl, i-propyl, fluorine, chlorine, bromine, methoxy or ethoxy,

Y represents hydrogen, methyl, ethyl, propyl, i-propyl, butyl, i-butyl, tert.-butyl, fluorine, chlorine, bromine, methoxy, ethoxy or trifluoromethyl,

Z represents methyl, ethyl, i-propyl, butyl, i-butyl, tert.-butyl, fluorine, chlorine, bromine, methoxy or ethoxy,

n represents a number from 0-3,

R represents the groups

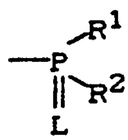 (a) $-SO_2-R^3$ (b)

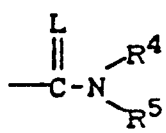 (c) or 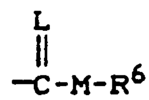 (d)

in which

L and M in each case represent oxygen or sulphur,

$R^1$, $R^2$ and $R^3$ independently of one another represent $C_1$-$C_4$-alkyl, $C_1$-$C_4$-alkoxy, $C_1$-$C_4$-alkylamino, di-($C_1$-$C_4$-alkyl)-amino or $C_1$-$C_4$-alkylthio, each of which is optionally substituted by fluorine or chlorine, or represent phenyl, benzyl, phenoxy or phenylthio, each of which is optionally substituted by fluorine, chlorine, bromine, nitro, cyano, $C_1$-$C_2$-alkoxy, $C_1$-$C_4$-fluoroalkoxy, $C_1$-$C_2$-chloroalkoxy, $C_1$-$C_2$-alkylthio, $C_1$-$C_2$-fluoroalkylthio, $C_1$-$C_2$-chloroalkylthio or $C_1$-$C_3$-alkyl,

$R^4$ and $R^5$ independently of one another represent $C_1$-$C_{10}$-alkyl, $C_1$-$C_{10}$-alkoxy or $C_1$-$C_{10}$-alkoxy-($C_1$-$C_{10}$)-alkyl, each of which is optionally substituted by fluorine, chlorine or bromine, or represent phenyl which is optionally substituted by fluorine, chlorine, bromine, $C_1$-$C_{20}$-halogenoalkyl, $C_1$-$C_{20}$-alkyl or $C_1$-$C_4$-alkoxy, or represent benzyl which is optionally substituted by fluorine, chlorine, bromine, $C_1$-$C_4$-alkyl, $C_1$-$C_4$-halogenoalkyl or $C_1$-$C_4$-alkoxy,

$R^6$ represents $C_1$-$C_{10}$-alkyl which is optionally substituted by fluorine, chlorine or bromine and which can be interrupted by oxygen, or represents phenyl which is optionally substituted by fluorine, chlorine, bromine, $C_1$-$C_4$-halogenoalkyl or $C_1$-$C_4$-alkoxy, or represents benzyl which is optionally substituted by fluorine, chlorine, bromine, $C_1$-$C_4$-halogenoalkyl or $C_1$-$C_4$-alkoxy,

as well as the pure enantiomeric forms of compounds of the formula (I), with the proviso that L and M

do not simultaneously represent oxygen.

4. Process for the preparation of 3-arylpyrrolidine-2,4-dione derivatives of the formula (I) according to Claim 1,
characterized in that, to obtain compounds of the structure (Ia)

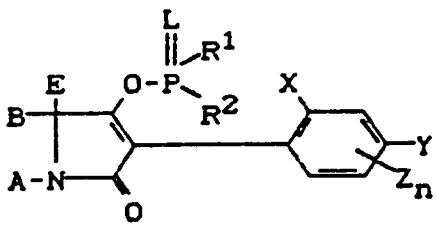

(Ia)

in which
A, B, E, L, X, Y, Z, $R^1$, $R^2$ and n have the meaning given in Claim 1, 3-aryl-pyrrolidine-2,4-diones of the formula (II) or their enols

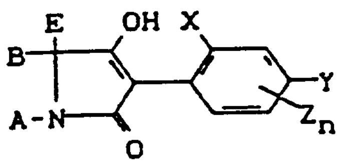

(II)

in which
A, B, E, X, Y, Z and n have the meaning given in Claim 1,
are reacted with phosphorus compounds of the general formula (III)

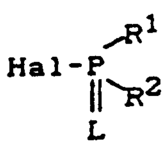

(III)

in which
L, $R^1$ and $R^2$ have the meaning given in Claim 1
and
Hal represents halogen,
if appropriate in the presence of a diluent and if appropriate in the presence of an acid-binding agent,
or in that, to obtain compounds of the formula (Ib)

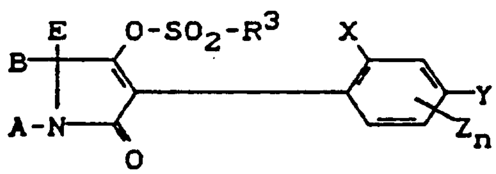

(Ib)

in which
A, B, E, X, Y, Z, $R^3$ and n have the meaning given in Claim 1,
compounds of the formula (II)

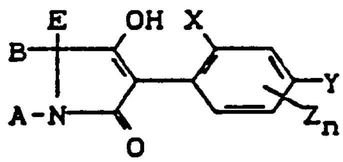

(II)

in which

A, B, E, X, Y, Z and n have the meaning given in Claim 1

are reacted with sulphonyl chlorides of the general formula (IV)

$R^3$-$SO_2$-Cl (IV)

in which

$R^3$ has the meaning given in Claim 1, if appropriate in the presence of a diluent and if appropriate in the presence of an acid-binding agent,

or in that, to obtain compounds of the formula (Ic)

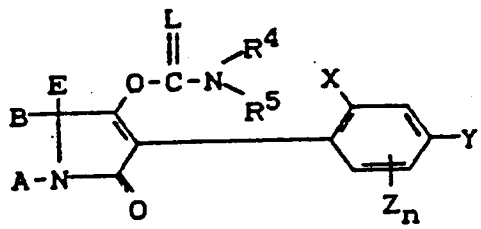

(Ic)

in which

A, B, E, L, X, Y, Z, $R^4$, $R^5$ and n have the meaning given in Claim 1,

compounds of the formula (II)

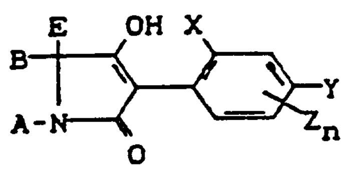

(II)

in which

A, B, E, X, Y, Z and n have the meaning given in Claim 1

are reacted

α) with isocyanates of the general formula (V)

$R^4$-N = C = O (V)

in which

$R^4$ has the meaning given in Claim 1,

if appropriate in the presence of a diluent and if appropriate in the presence of a catalyst,

or

β) with carbamoyl chlorides or thiocarbamoyl chlorides of the general formula (VI)

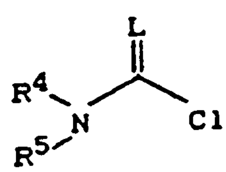

(VI)

in which
L, $R^4$ and $R^5$ have the meaning given in Claim 1,
if appropriate in the presence of a diluent and if appropriate in the presence of an acid-binding agent,
or in that, to obtain compounds of the formula (Id)

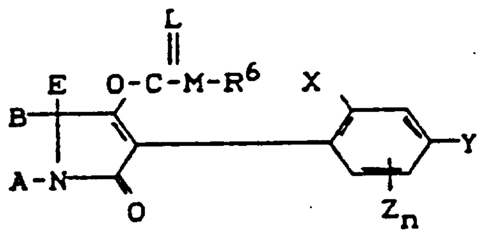

(Id)

in which
A, B, E, L, M, $R^6$, X, Y, Z and n have the meaning given in Claim 1,
compounds of the formula (II)

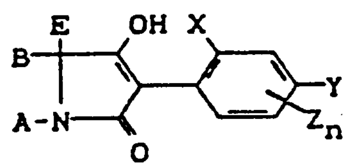

(II)

in which
A, B, E, X, Y, Z and n have the meaning given in Claim 1
are reacted
α) with chloromonothioformic eaters or chlorodithioformic esters of the general formula VII

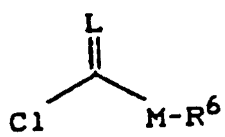

(VII)

in which
L, M and $R^6$ have the meaning given in Claim 1,
if appropriate in the presence of a diluent and if appropriate in the presence of an acid-binding agent,
or
β) with carbon disulphide and subsequently with alkyl halides of the general formula VIII

$R^6$-Hal (VIII)

in which
$R^6$ has the meaning given in Claim 1
and
Hal represents chlorine, bromine or iodine,
or in that, to obtain compounds of the formula (Ie)

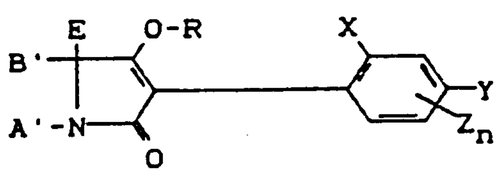

(Ie)

in which
R, X, Y, Z and n have the meaning given in Claim 1,
E represents hydrogen
and
A' and B' together represent $-CH_2-CH_2-SO-CH_2-$, $-CH_2-SO-CH_2-CH_2-$ or $CH_2-SO-CH_2-$,
compounds of the formula (If)

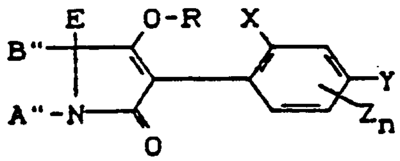

(If)

in which
R, X, Y, Z and n have the meaning given in Claim 1,
E represents hydrogen
and
A" and B" together represent $-CH_2-CH_2-S-CH_2-$, $-CH_2-S-CH_2-CH_2-$ or $-CH_2-S-CH_2-$,
are reacted with approximately equimolar amounts of an oxidant, if appropriate in the presence of a diluent,
or in that, to obtain compounds of the formula (Ig)

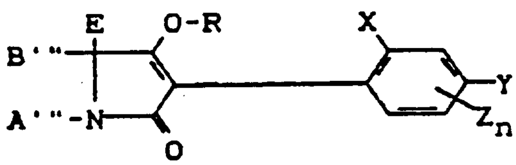

(Ig)

in which
R, X, Y, Z and n have the meaning given in Claim 1,
E represents hydrogen
and
A''' and B''' together represent $-CH_2-CH_2-SO_2-CH_2-$, $-CH_2-SO_2-CH_2-CH_2-$ or $-CH_2-SO_2-CH_2-$,
compounds of the formula (If)

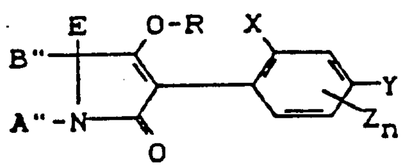

(If)

in which
R, X, Y, Z and n have the meaning given in Claim 1,
E represents hydrogen
and
A" and B" together represent $-CH_2-CH_2-S-CH_2-$, $-CH_2-S-CH_2-CH_2-$ and $-CH_2-S-CH_2-$,
are reacted with at least twice the equimolar amounts of an oxidant, if appropriate in the presence of a diluent.

5. Insecticidal, acaricidal and herbicidal agents, characterized in that they contain at least one 3-aryl-pyrrolidine-2,4-dione derivative of the formula (I).

6. Method of combating insects and/or arachnids and/or weeds, characterized in that 3-arylpyrrolidine-2,4-dione derivatives of the formula (I) are allowed to act on insects and/or arachnids and/or weeds and/or their environment.

7. Use of 3-arylpyrrolidine-2,4-dione derivatives of the formula (I) for combating insects and/or arachnids and/or weeds.

8. Process for the preparation of insecticidal and/or acaricidal and/or herbicidal agents, characterized in that 3-arylpyrrolidine-2,4-dione derivatives of the formula (I) are mixed with extenders and/or surface-active agents.

**Revendications**

1. Dérivés de 3-aryl-pyrrolidine-2,4-diones répondant à la formule (I)

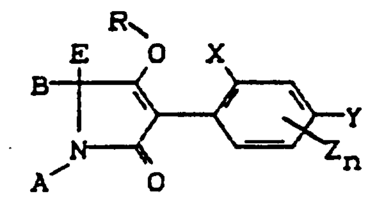

(I)

dans laquelle

- A représente un groupe alkyle en $C_1$-$C_{12}$ à chaîne droite ou ramifiée portant éventuellement un ou plusieurs substituants halogéno identiques ou différents, un groupe alcényle en $C_3$-$C_8$, un groupe alcynyle en $C_3$-$C_8$, un groupe alcoxy(en $C_1$-$C_{10}$)alkyle en $C_2$-$C_8$, un groupe polyalcoxy(en $C_1$-$C_8$)alkyle en $C_2$-$C_8$, un groupe alkyl(en $C_1$-$C_{10}$)thioalkyle en $C_2$-$C_8$, un groupe cycloalkyle contenant 3 à 8 atomes cycliques, qui peut être interrompu par un atome d'oxygène et/ou par un atome de soufre, ou encore un groupe aryl(alkyle en $C_1$-$C_6$) qui porte éventuellement un ou plusieurs substituants identiques ou différents halogéno, alkyl en $C_1$-$C_6$,halogénalkyle en $C_1$-$C_6$, alcoxy en $C_1$-$C_6$, nitro,
- B et E représentent, chacun indépendamment l'un de l'autre, un atome d'hydrogène, un groupe alkyle en $C_1$-$C_{12}$ à chaîne droite ou ramifiée, un groupe alcoxyalkyle en $C_1$-$C_8$,

ou bien

- A et B forment, avec l'atome d'azote respectivement l'atome de carbone auquel ils sont liés, un cycle tétra- à octogonal, qui peut être interrompu par un atome de soufre, par un groupe sulfoxyde ou par un groupe sulfonyle,
- X représente un groupe alkyle en $C_1$-$C_6$, un atome d'halogène ou un groupe alcoxy en $C_1$-$C_6$,
- Y représente un atome d'hydrogène, un groupe alkyle en $C_1$-$C_6$, un atome d'halogène, un groupe alcoxy en $C_1$-$C_6$, un groupe halogénalkyle en $C_1$-$C_3$,
- Z représente un groupe alkyle en $C_1$-$C_6$, un atome d'halogène, un groupe alcoxy en $C_1$-$C_6$,
- n représente un nombre de 0 à 3,
- R représente les groupes

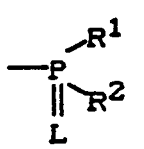

(a)

$-SO_2-R^3$ (b)

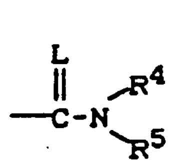

(c) ou

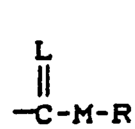

(d)

dans lesquels

- L et M représentent respectivement un atome d'oxygène ou atome de soufre,
- $R^1$, $R^2$ et $R^3$ représentent, chacun indépendamment l'un de l'autre, un groupe alkyle en $C_1$-$C_8$

portant éventuellement un ou plusieurs substituants halogéno identiques ou différents, un groupe alcoxy en $C_1$-$C_8$, un groupe alkyl(en $C_1$-$C_8$)amino, un groupe dialkyl(en $C_1$-$C_8$)amino, un groupe alkyl(en $C_1$-$C_8$)thio, un groupe alcényl(en $C_2$-$C_5$)-thio, un groupe alcynyl(en $C_2$-$C_5$)thio, un groupe cycloalkyl(en $C_3$-$C_7$)thio; un groupe benzyle, un groupe phénoxy, un groupe phénylthio ou un groupe phényle portant éventuellement un ou plusieurs substituants identiques ou différents halogéno, nitro, cyano, alcoxy en $C_1$-$C_4$, halogénalcoxy en $C_1$-$C_4$, alkyl(en $C_1$-$C_4$)thio, halogénalkyl-(en $C_1$-$C_4$)thio, alkyle en $C_1$-$C_4$, halogénalkyle en $C_1$-$C_4$,

$R^4$ et $R^5$ représentent, chacun indépendamment l'un de l'autre, un groupe alkyle en $C_1$-$C_{20}$ portant éventuellement un ou plusieurs substituants halogéno identiques ou différents, un groupe alcoxy en $C_1$-$C_{20}$, un groupe alcényle en $C_2$-$C_8$, un groupe alcoxy-(en $C_1$-$C_{20}$)alkyle en $C_1$-$C_{20}$; un groupe phényle portant éventuellement un ou plusieurs substituants identiques ou différents halogéno, halogénalkyle en $C_1$-$C_{20}$, alkyle en $C_1$-$C_{20}$ ou alcoxy en $C_1$-$C_{20}$; un groupe benzyle portant éventuellement un ou plusieurs substituants identiques ou différents halogéno, alkyle en $C_1$-$C_{20}$, halogénalkyle en $C_1$-$C_{20}$ ou alcoxy en $C_1$-$C_{20}$; ou encore représentent ensemble un noyau alkylène en $C_2$-$C_6$ éventuellement interrompu par un atome d'oxygène,

$R_6$ représente un groupe alkyle en $C_1$-$C_{20}$ portant éventuellement un ou plusieurs substituants halogéno identiques ou différents, qui peut être interrompu par un atome d'oxygène; un groupe phényle portant éventuellement un ou plusieurs substituants identiques ou différents halogéno, halogénalkyle en $C_1$-$C_{20}$, alcoxy en $C_1$-$C_{20}$; un groupe benzyle portant éventuellement un ou plusieurs substituants identiques ou différents halogéno, halogénalkyle en $C_1$-$C_{20}$ ou alcoxy en $C_1$-$C_{20}$; un groupe alcényle en $C_2$-$C_8$ ou encore un groupe alcynyle en $C_2$-$C_5$,

ainsi que les formes pures des énantiomères des composés de formule (I), avec cette mesure que L et M ne représentent pas simultanément un atome d'oxygène.

**2.** Dérivés de 3-aryl-pyrrolidine-2,4-diones de formule (I) selon la revendication 1, dans laquelle

A représente un groupe alkyle en $C_1$-$C_{10}$ à chaîne droite ou ramifiée portant éventuellement un ou plusieurs substituants halogéno identiques ou différents, un groupe alcényle en $C_3$-$C_6$, un groupe alcynyle en $C_3$-$C_6$, un groupe alcoxy(en $C_1$-$C_8$)alkyle en $C_2$-$C_8$, un groupe polyalcoxy(en $C_1$-$C_6$)alkyle en $C_2$-$C_6$, un groupe alkyl(en $C_1$-$C_8$)thioalkyle en $C_2$-$C_6$, un groupe cycloalkyle contenant 3 à 7 atomes cycliques, qui peut être interrompu par 1-2 atomes d'oxygène et/ou de soufre, ou encore un groupe aryl(alkyle en $C_1$-$C_4$) qui porte éventuellement un ou plusieurs substituants identiques ou différents halogéno, alkyle en $C_1$-$C_4$, halogénalkylen $C_1$-$C_4$,alcoxy en $C_1$-$C_4$, nitro,

B, E représentent, chacun indépendamment l'un de l'autre, un atome d'hydrogène, un groupe alkyle en $C_1$-$C_{10}$ à chaîne droite ou ramifiée, un groupe alcoxyalkyle en $C_1$-$C_6$,

ou bien

A et B forment, avec l'atome d'azote respectivement l'atome de carbone auquel ils sont liés, un cycle tétra- à heptagonal, qui peut être interrompu par un atome de soufre, par un groupe sulfoxyde ou par un groupe sulfonyle,

X représente un groupe alkyle en $C_1$-$C_4$, un atome d'halogène ou un groupe alcoxy en $C_1$-$C_4$,

Y représente un atome d'hydrogène, un groupe alkyle en $C_1$-$C_6$, un atome d'halogène, un groupe alcoxy en $C_1$-$C_4$, un groupe halogénalkyle en $C_1$-$C_2$,

Z représente un groupe alkyle en $C_1$-$C_4$, un atome d'halogène, un groupe alcoxy en $C_1$-$C_4$,

n représente un nombre de 0 à 3,

R représente les groupes

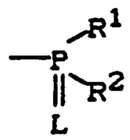 (a) $-SO_2-R^3$ (b) .

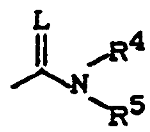 (c) 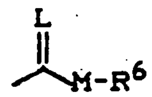 (d)

dans lesquels

- L et M représentent respectivement un atome d'oxygène ou atome de soufre,
- $R^1$, $R^2$ et $R^3$ représentent, chacun indépendamment l'un de l'autre, un groupe alkyle en $C_1$-$C_6$ portant éventuellement un ou plusieurs substituants halogéno identiques ou différents, un groupe alcoxy en $C_1$-$C_6$, un groupe alkyl(en $C_1$-$C_6$)amino, un groupe dialkyl(en $C_1$-$C_6$)amino, un groupe alkyl(en $C_1$-$C_6$)thio, un groupe alcényl(en $C_3$-$C_4$)-thio, un groupe alcynyl(en $C_2$-$C_4$)thio, un groupe cycloalkyl(en $C_3$-$C_6$)thio; un groupe benzyle, un groupe phénoxy, un groupe phénylthio ou un groupe phényle portant éventuellement un ou plusieurs substituants identiques ou différents fluoro, chloro, bromo, nitro, cyano, alcoxy en $C_1$-$C_3$, halogénalcoxy en $C_1$-$C_3$, alkyl(en $C_1$-$C_3$)thio, halogénalkyl(en $C_1$-$C_3$)thio, alkyle en $C_1$-$C_3$, halogénalkyle en $C_1$-$C_3$,
- $R^4$ et $R^5$ représentent, chacun indépendamment l'un de l'autre, un groupe alkyle en $C_1$-$C_{20}$ portant éventuellement un ou plusieurs substituants halogéno identiques ou différents, un groupe alcoxy en $C_1$-$C_{20}$, un groupe alcényle en $C_2$-$C_8$, un groupe alcoxy-(en $C_1$-$C_{20}$)alkyle en $C_1$-$C_{20}$; un groupe phényle portant éventuellement un ou plusieurs substituants identiques ou différents halogéno, halogénalkyle en $C_1$-$C_5$, alkyle en $C_1$-$C_5$ ou alcoxy en $C_1$-$C_5$; un groupe benzyle portant éventuellement un ou plusieurs substituants identiques ou différents halogéno, alkyle en $C_1$-$C_5$, halogénalkyle en $C_1$-$C_5$ ou alcoxy en $C_1$-$C_5$;
- $R_6$ représente un groupe alkyle en $C_1$-$C_{20}$ portant éventuellement un ou plusieurs substituants halogéno identiques ou différents, qui peut être interrompu par un atome d'oxygène; un groupe phényle portant éventuellement un ou plusieurs substituants identiques ou différents halogéno, halogénalkyle en $C_1$-$C_5$ ou alcoxy en $C_1$-$C_5$; ou encore un groupe benzyle portant éventuellement un ou plusieurs substituants identiques ou différents halogéno, halogénalkyle en $C_1$-$C_5$ ou alcoxy en $C_1$-$C_5$,

ainsi que les formes pures des énantiomères des composés de formule (I), avec cette mesure que L et M ne représentent pas simultanément un atome d'oxygène.

**3.** Dérivés de 3-aryl-pyrrolidine-2,4-diones de formule (I) selon la revendication 1, dans laquelle

- A représente un groupe alkyle en $C_1$-$C_8$ à chaîne droite ou ramifiée portant éventuellement un ou plusieurs substituants halogéno identiques ou différents, un groupe alcényle en $C_3$-$C_4$, un groupe alcynyle en $C_3$-$C_4$, un groupe alcoxy(en $C_1$-$C_6$)alkyle en $C_2$-$C_4$, un groupe polyalcoxy(en $C_1$-$C_4$)alkyle en $C_2$-$C_4$, un groupe alkyl(en $C_1$-$C_6$)thioalkyle en $C_2$-$C_4$, un groupe cycloalkyle contenant 3 à 6 atomes cycliques, qui peut être interrompu par 1-2 atomes d'oxygène et/ou de soufre, ou encore un groupe aryl(alkyle en $C_1$-$C_3$) qui porte éventuellement un ou plusieurs substituants identiques ou différents fluoro, chloro, bromo, méthyle, éthyle, propyle, isopropyle, méthoxy, éthoxy, trifluorométhyle, nitro,
- B, E représentent, chacun indépendamment l'un de l'autre, un atome d'hydrogène, un groupe alkyle en $C_1$-$C_8$ à chaîne droite ou ramifiée, un groupe alcoxyalkyle en $C_1$-$C_4$,

ou bien

- A et B forment, avec l'atome d'azote respectivement l'atome de carbone auquel ils sont liés, un cycle penta- à hexagonal, qui peut être interrompu par un atome de soufre, par un groupe sulfoxyde ou par un groupe sulfonyle,

X représente un groupe méthyle, un groupe éthyle, un groupe propyle, un groupe i-propyle, un atome de fluor, un atome de chlore, un atome de brome, un groupe méthoxy ou un groupe éthoxy,

Y représente un atome d'hydrogène, un groupe méthyle, un groupe éthyle, un groupe propyle, un groupe i-propyle, un groupe butyle, un groupe i-butyle, un groupe tert.-butyle, un atome de fluor, un atome de chlore, un atome de brome, un groupe méthoxy, un groupe éthoxy ou un groupe trifluorométhyle,

Z représente un groupe méthyle, un groupe éthyle, un groupe i-propyle, un groupe butyle, un groupe i-butyle, un groupe tert.-butyle, un atome de fluor, un atome de chlore, un atome de brome, un groupe méthoxy ou un groupe éthoxy,

n représente un nombre de 0 à 3,

R représente les groupes

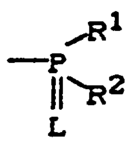 (a) $-SO_2-R^3$ (b)

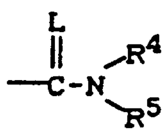 (c) ou 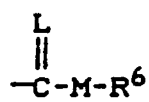 (d)

dans lesquels

L et M représentent respectivement un atome d'oxygène ou atome de soufre,

$R^1$, $R^2$ et $R^3$ représentent, chacun indépendamment l'un de l'autre, un groupe alkyle en $C_1$-$C_4$ portant éventuellement un ou plusieurs substituants fluoro ou chloro identiques ou différents, un groupe alcoxy en $C_1$-$C_4$, un groupe alkyl(en $C_1$-$C_4$)amino, un groupe dialkyl(en $C_1$-$C_4$)amino, un groupe alkyl(en $C_1$-$C_4$)thio; un groupe benzyle, un groupe phénoxy, un groupe phénylthio ou un groupe phényle portant éventuellement un ou plusieurs substituants identiques ou différents fluoro, chloro, bromo, nitro, cyano, alcoxy en $C_1$-$C_2$, fluoralcoxy en $C_1$-$C_4$, chloralcoxy en $C_1$-$C_2$, alkyl(en $C_1$-$C_2$)thio, fluoralkyl(en $C_1$-$C_2$)thio, chloralkyl(en $C_1$-$C_2$)thio, alkyle en $C_1$-$C_3$,

$R^4$ et $R^5$ représentent, chacun indépendamment l'un de l'autre, un groupe alkyle en $C_1$-$C_{10}$ portant éventuellement un ou plusieurs substituants fluoro, chloro, bromo identiques ou différents, un groupe alcoxy en $C_1$-$C_{10}$, un groupe alcoxy(en $C_1$-$C_{10}$)alkyle en $C_1$-$C_{10}$; un groupe phényle portant éventuellement un ou plusieurs substituants identiques ou différents fluoro, chloro, bromo, halogénalkyle en $C_1$-$C_{20}$, alkyle en $C_1$-$C_{20}$ ou alcoxy en $C_1$-$C_4$; un groupe benzyle portant éventuellement un ou plusieurs substituants identiques ou différents fluoro, chloro, bromo, alkyle en $C_1$-$C_4$, halogénalkyle en $C_1$-$C_4$ ou alcoxy en $C_1$-$C_4$,

$R_6$ représente un groupe alkyle en $C_1$-$C_{10}$ portant éventuellement un ou plusieurs substituants identiques ou différents fluoro, chloro, bromo, qui peut être interrompu par un atome d'oxygène; un groupe phényle portant éventuellement un ou plusieurs substituants identiques ou différents fluoro, chloro, bromo, un groupe halogénalkyle en $C_1$-$C_4$, alcoxy en $C_1$-$C_4$; ou encore un groupe benzyle portant éventuellement un ou plusieurs substituants identiques ou différents fluoro, chloro, bromo, halogénalkyle en $C_1$-$C_4$ ou alcoxy en $C_1$-$C_4$,

ainsi que les formes pures des énantiomères des composés de formule (I), avec cette mesure que L et M ne représentent pas simultanément un atome d'oxygène.

**4.** Procédé pour la préparation de dérivés de 3-arylpyrrolidin-2,4-diones de formule (I) selon la revendication 1

caractérisé en ce que, pour obtenir des composés de structure (Ia)

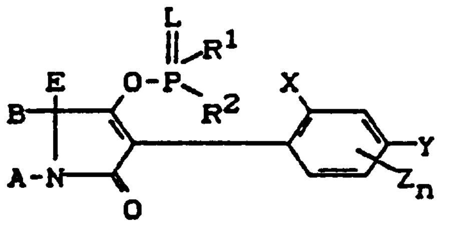

(Ia)

dans laquelle

A, B, E, L, X, Y, Z, $R^1$ et $R^2$ et n ont la signification indiquée à la revendication 1,

on fait réagir des 3-aryl-pyrrolidine-2,4-diones de formule (II), respectivement leurs énols

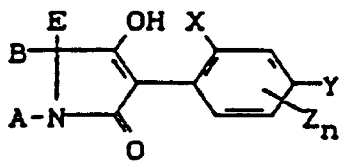

(II)

dans laquelle

A, B, E, X, Y, Z et n ont la signification indiquée à la revendication 1,

avec des composés phosphorés répondant à la formule générale (III)

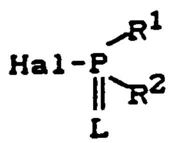

(III)

dans laquelle

L, $R^1$ et $R^2$ ont la signification indiquée à la revendication 1,

et

Hal représente un atome d'halogène,

éventuellement en présence d'un diluant et éventuellement en présence d'un agent neutralisateur d'acides,

ou bien en ce que, pour obtenir des composés de formule (Ib),

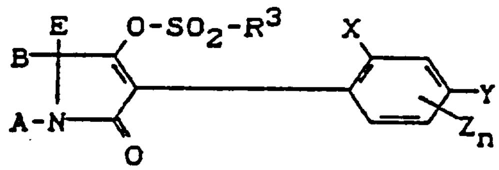

(Ib)

dans laquelle

A, B, E, X, Y, Z, $R^3$ et n ont la signification indiquée à la revendication 1,

on fait réagir des composés de formule (II)

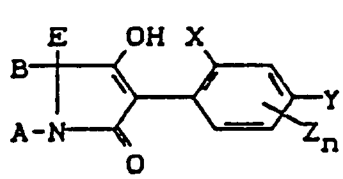

(II)

dans laquelle

A, B, E, X, Y, Z et n ont la signification indiquée à la revendication 1,

avec des chlorures d'acides sulfoniques répondant à la formule générale (IV)

$R^3-SO_2-Cl$ (IV)

dans laquelle

$R^3$ a la signification indiquée à la revendication 1,

éventuellement en présence d'un diluant et éventuellement en présence d'un agent neutralisateur d'acides,

ou bien en ce que, pour obtenir des composés de formule (Ic)

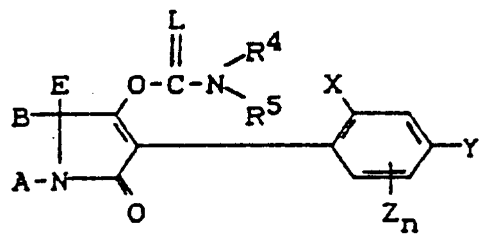

(Ic)

dans laquelle

A, B, E, L, X, Y, Z, $R^4$ et $R^5$ et n ont la signification indiquée à la revendication 1,

on fait réagir des composés de formule (II)

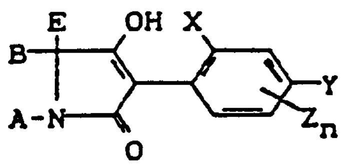

(II)

dans laquelle

A, B, E, X, Y, Z et n ont la signification indiquée à la revendication 1,

$\alpha$) avec des isocyanates répondant à la formule générale (V)

$R^4-N=C=O$ (V)

dans laquelle

$R^4$ a la signification indiquée à la revendication 1,

éventuellement en présence d'un diluant et éventuellement en présence d'un catalyseur,

ou

$\beta$) avec des chlorures d'acide carbamique ou avec des chlorures d'acide thiocarbamique répondant à la formule générale (VI)

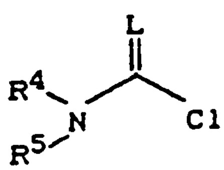

(VI)

dans laquelle

L, $R^4$ et $R^5$ ont la signification indiquée à la revendication 1,

éventuellement en présence d'un diluant et éventuellement en présence d'un agent neutralisateur d'acides,

ou bien en ce que, pour obtenir des composés de formule (Id)

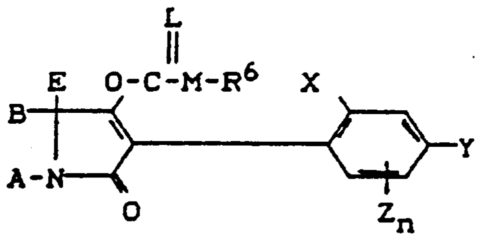

(Id)

dans laquelle

A, B, E, L, M, $R^6$, X, Y, Z et n ont la signification indiquée à la revendication 1,

on fait réagir des composés de formule (II)

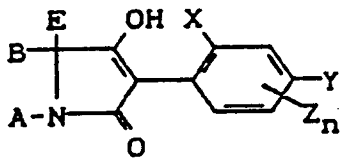

(II)

dans laquelle

A, B, E, X, Y, Z et n ont la signification indiquée à la revendication 1,

α) avec des esters chloromonothioformiques ou avec des esters chlorodithioformiques répondant à la formule générale (VII)

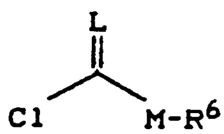

(VII)

dans laquelle

L, M, $R^6$ ont la signification indiquée à la revendication 1,

éventuellement en présence d'un diluant et éventuellement en présence d'un agent neutralisateur d'acides, ou

β) avec du sulfure de carbone et ensuite, avec des halogénures d'alkyle répondant à la formule générale (VIII)

$R^6$-HAl (VIII)

dans laquelle

$R^6$ a la signification indiquée à la revendication 1,

et

Hal représente un atome de chlore, un atome de brome, un atome d'iode,

ou bien en ce que, pour obtenir des composés de formule (Ie),

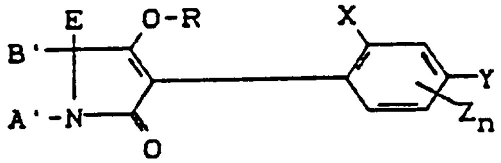

(Ie)

dans laquelle

R, X, Y, Z et n ont la signification indiquée à la revendication 1,

E représente un atome d'hydrogène

et

A', B' représentent ensemble $-CH_2-CH_2-SO-CH_2-$, $-CH_2-SO-CH_2-CH_2-$ ou $-CH_2-SO-CH_2-$,

on fait réagir des composés de formule (If)

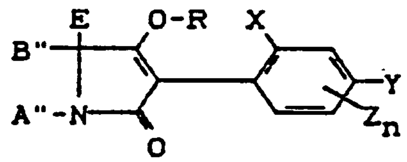

, (If)

dans laquelle

R, X, Y, Z et n ont la signification indiquée à la revendication 1,

E représente un atome d'hydrogène

et

A'', B'' représentent ensemble $-CH_2-CH_2-S-CH_2-$, $-CH_2-S-CH_2-CH_2-$ ou $-CH_2-S-CH_2-$,

avec des quantités approximativement équimolaires d'un agent d'oxydation éventuellement en présence d'un diluant,

ou bien en ce que, pour obtenir des composés de formule (Ig),

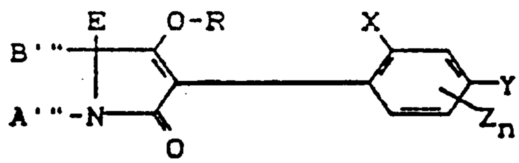

(Ig)

dans laquelle

R, X, Y, Z et n ont la signification indiquée à la revendication 1,

E représente un atome d'hydrogène

et

A''', B''' représentent ensemble $-CH_2-CH_2-SO_2-CH_2-$, $-CH_2-SO_2-CH_2-CH_2-$ ou $-CH_2-SO_2-CH_2-$,

on fait réagir des composés de formule (If)

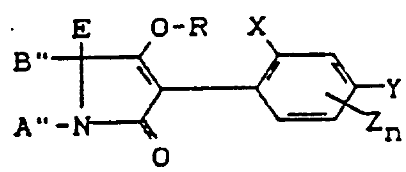

(If)

dans laquelle

R, X, Y, Z et n ont la signification indiquée à la revendication 1,

E représente un atome d'hydrogène

et

A'', B'' représentent ensemble $-CH_2-CH_2-S-CH_2-$, $-CH_2-S-CH_2-CH_2-$ et $-CH_2-S-CH_2-$,

avec des quantités au moins deux fois équimolaires d'un agent d'oxydation, éventuellement en présence d'un diluant.

**5.** Insecticides, acaricides et herbicides caractérisés par une teneur en au moins un dérivé de 3-aryl-pyrrolidine-2,4-dione de formule (I).

**6.** Procédé pour lutter contre les insectes et/ou contre les arachnides et/ou contre les mauvaises herbes, caractérisé en ce qu'on laisse agir des dérivés de 3-aryl-pyrrolidine-2,4-diones de formule (I) sur des insectes et/ou sur des arachnides et/ou sur des mauvaises herbes et/ou sur leur biotope.

**7.** Utilisation de dérivés de 3-aryl-pyrrolidine-2,4-diones de formule (I) pour lutter contre les insectes et/ou les arachnides et/ou les mauvaises herbes.

**8.** Procédé pour la préparation d'insecticides et/ou d'acaricides et/ou d'herbicides, caractérisé en ce qu'on mélange des dérivés de 3-aryl-pyrrolidine-2,4-diones de formule (I) avec des diluants et/ou avec des agents tensioactifs.